(19) Europäisches Patentamt
European Patent Office
Office européen des brevets

(11) Veröffentlichungsnummer: **0 272 588 B1**

(12) **EUROPÄISCHE PATENTSCHRIFT**

(45) Veröffentlichungstag der Patentschrift: **14.10.92**

(51) Int. Cl.$^5$: **C08K 5/34**, C07D 487/22, C09D 7/12

(21) Anmeldenummer: **87118605.2**

(22) Anmeldetag: **15.12.87**

(54) **Acetylendiharndstoffderivate und ihre Verwendung zum Stabilisieren von Kunststoffen.**

(30) Priorität: **22.12.86 DE 3643889**

(43) Veröffentlichungstag der Anmeldung:
**29.06.88 Patentblatt 88/26**

(45) Bekanntmachung des Hinweises auf die Patenterteilung:
**14.10.92 Patentblatt 92/42**

(84) Benannte Vertragsstaaten:
**BE CH DE FR GB IT LI NL**

(56) Entgegenhaltungen:
**EP-A- 0 001 284**
**EP-A- 0 213 570**
**DE-A- 3 236 070**
**FR-A- 2 291 203**

**Chemical Abstracts, Vol. 103, 29. Juli - 12. August 1985, Columbus, Ohio, USA, MASUI et al. "N-Cyanomethylation of 2,2,6,6-tetramethylpiperidines by anodic oxidation in acetonitrile" Seite 500, Zusammenfassung-Nr. 37 343k & J. Chem.Soc., Chem. Commun. 1985, (5), Seiten 272-273**

(73) Patentinhaber: **BASF Aktiengesellschaft**
**Carl-Bosch-Strasse 38**
**W-6700 Ludwigshafen(DE)**

(72) Erfinder: **Aumueller, Alexander, Dr.**
**An der Marlach 5**
**W-6705 Deidesheim(DE)**
Erfinder: **Neumann, Peter, Dr.**
**Franz-Schubert-Strasse 1**
**W-6908 Wiesloch(DE)**
Erfinder: **Trauth, Hubert**
**Milanstrasse 5**
**W-6724 Dudenhofen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 272 588 B1

Rank Xerox (UK) Business Services

## Beschreibung

Es ist bekannt, daß 2,2,6,6-Tetraalkyl-piperidinderivate Lichtschutzmittel für organische Polymere sind. In der nicht vorveröffentlichten EP-A 213 570 werden Acetylendiharnstoffderivate mit eingebauten 2,2,6,6-Tetraalkylpiperidinresten beschrieben. Es ist ebenfalls bekannt, daß Harnstoff- und Thioharnstoffderivate eine gute Wärmestabilisierung in einigen Kunststoffen erzielen. Jedoch besitzen nur 4-Hydroxyphenyl- oder 4-Alkoxyphenylsubstituierte (Thio-)Harnstoffe eine allgemeine UV-Absorberwirkung (vgl. J. Voigt, in K.A. Wolf (Hrsg.), Chemie, Physik und Technologie der Kunststoffe, Springer-Verlag, Berlin, Heidelberg, New York, 1966, S. 300-303).

In der FR-A-2 291 203 sind Glycolurilderivate der Formel

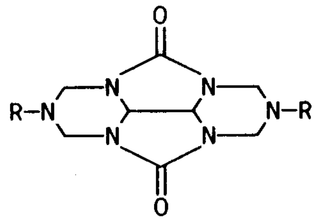

beschrieben, worin R einen gegebenenfalls substituierten Kohlenwasserstoffrest mit bis zu 22 Kohlenstoffatomen bedeutet. Diese Verbindungen werden als oberflächenaktive Mittel für die Textilindustrie und als Korrosionsinhibitoren vorgeschlagen.

Der Erfindung liegt die Aufgabe zugrunde, neue oder bekannte, aber bisher für diese Anwendung noch nicht vorgeschlagene Verbindungen als Stabilisatoren für Kunststoffe und Lacke bereitzustellen.

Diese Aufgabe wird gelöst durch die Verwendung von Verbindungen der allgemeinen Formel (I)

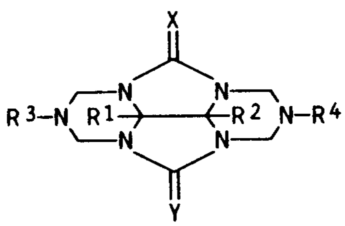

(I),

worin

$R^1$ und $R^2$ unabhängig voneinander Wasserstoff, $C_1$-$C_8$-Alkyl, $C_5$-$C_8$-Cycloalkyl, $C_7$-$C_{12}$-Aralkyl, $C_7$-$C_{14}$-Cycloalkylalkyl, Aryl oder Carbonester bedeuten oder $R^1$ und $R^2$ zusammen eine Tetra-, Penta- oder Hexamethylengruppierung oder einen gegebenenfalls substituierten Rest der Formel

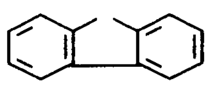

darstellen,

X und Y unabhängig voneinander für Sauerstoff, Schwefel oder $NR^8$ stehen, wobei $R^8$ Wasserstoff, $C_1$-$C_8$-Alkyl oder $C_7$-$C_{12}$-Aralkyl ist, und

$R^3$ und $R^4$ unabhängig voneinander für $C_1$-$C_{22}$-Alkyl, das durch Ethersauerstoff, Schwefel oder Stickstoff unterbrochen sein kann, $C_1$-$C_{22}$-Alkenyl, $C_3$-$C_{12}$-Cycloalkyl, gegebenenfalls substituiertes $C_7$-$C_{12}$-Aralkyl, Hydroxy, $C_3$-$C_{12}$-Alkinyl, einen nichtaromatischen, gegebenenfalls substituierten Heterocyclus, ausgenommen 2,2,6,6-Tetraalkylpiperidin für den Fall, daß $R^3$ gleich $R^4$ ist, durch einen Heterocyclus substituiertes $C_1$-$C_{22}$-Alkyl, ausgenommen 2,2,6,6-Tetraalkylpiperidin, für den Fall, daß $R^3$ gleich $R^4$ ist, durch Amino, Hydroxy, Carboxyl, Carbonester und/oder gegebenenfalls substituiertes Carbamoyl substituiertes $C_1$-$C_{22}$-Alkyl, oder für die Gruppierung B-A- stehen, worin -A- ein Brückenglied und B- einen Thiol- oder

Cyanrest oder eine Gruppe der Formeln

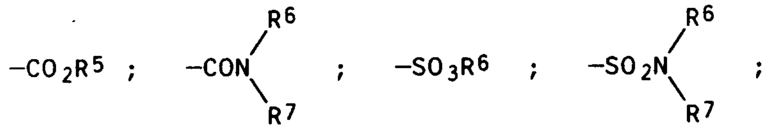

bedeuten, worin
$R^5$ für Wasserstoff, $C_1$-$C_{22}$-Alkyl, $C_5$-$C_8$-Cycloalkyl, $C_7$-$C_{12}$-Cycloalkylalkyl, $C_7$-$C_{12}$-Aralkyl, gegebenenfalls substituiertes Aryl, einen Heterocyclus oder einen Heterocyclus enthaltendes $C_1$-$C_{22}$-Alkyl steht und
$R^6$ und $R^7$ unabhängig voneinander Wasserstoff, $C_1$-$C_{22}$-Alkyl, Ethersauerstoff enthaltendes $C_2$-$C_9$-Alkyl, $C_7$-$C_{12}$-Aralkyl, gegebenenfalls substituiertes Aryl, einen Heterocyclus oder einen Heterocyclus enthaltendes $C_1$-$C_{22}$-Alkyl darstellen oder $R^6$ und $R^7$ gemeinsam mit dem oder den Atom(en), an das (die) sie gebunden sind, ein 3 bis 12-gliedriges Ringsystem bilden,

sowie der Säureadditionssalze und Hydrate dieser Verbindungen, zum Stabilisieren von Kunststoffen und Lacken.

Bevorzugt ist die Verwendung der Verbindungen zum Stabilisieren von Polyolefinen, Polyamiden, Polyurethanen und Lacken.

Eine besonders bevorzugte Verwendung ist die zum Licht- und Wärmeschutz und als Metallionendesaktivator, insbesondere bei Kunststoffen.

Gegenstand der Erfindung sind ferner die neuen Verbindungen der allgemeinen Formel (Ib)

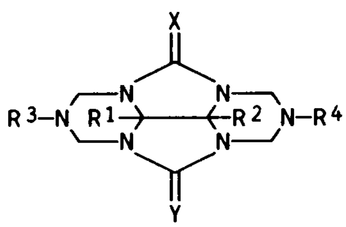

(Ib),

worin

- $R^1$ und $R^2$ unabhängig voneinander Methyl oder Phenyl bedeuten und einer der Reste $R^1$ oder $R^2$ auch für Wasserstoff stehen kann oder, wenn X und Y nicht gleichzeitig für Sauerstoff stehen, $R^1$ und $R^2$ auch gleichzeitig Wasserstoff bedeuten können,
- X und Y unabhängig voneinander für Sauerstoff, Schwefel oder $NR^8$ stehen, wobei $R^8$ Wasserstoff, $C_1$-$C_8$-Alkyl oder $C_7$-$C_{12}$-Aralkyl ist, und
- $R^3$ und $R^4$ unabhängig voneinander für $C_1$-$C_{22}$-Alkyl, das durch Ethersauerstoff, Schwefel oder Stickstoff unterbrochen sein kann, $C_1$-$C_{22}$-Alkenyl, $C_3$-$C_{12}$-Cycloalkyl, gegebenenfalls substituiertes $C_7$-$C_{12}$-Aralkyl, Hydroxy, $C_3$-$C_{12}$-Alkinyl, einen nichtaromatischen, gegebenenfalls substituierten Heterocyclus, ausgenommen 2,2,6,6-Tetraalkylpiperidin für den Fall, daß $R^3$ gleich $R^4$, durch einen Heterocyclus substituiertes $C_1$-$C_{22}$-Alkyl, ausgenommen 2,2,6,6-Tetraalkylpiperidin, für den Fall, daß $R^3$ gleich $R^4$, durch Amino, Hydroxy, Carboxyl, Carbonester und/oder gegebenenfalls substituiertes Carbamoyl substituiertes $C_1$-$C_{22}$-Alkyl, oder für die Gruppierung B-A- stehen, worin -A- ein Brückenglied und B- einen Thiol- oder Cyanrest oder eine Gruppe der Formeln

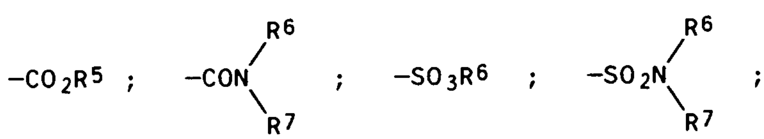

bedeutet, worin

$R^5$ für Wasserstoff, $C_1$-$C_{22}$-Alkyl, $C_5$-$C_8$-Cycloalkyl, $C_7$-$C_{12}$-Cycloalkylalkyl, $C_7$-$C_{12}$-Aralkyl, gegebenenfalls substituiertes Aryl, einen Heterocyclus oder einen Heterocyclus enthaltendes $C_1$-$C_{22}$-Alkyl steht und

$R^6$ und $R^7$ unabhängig voneinander Wasserstoff, $C_1$-$C_{22}$-Alkyl, Ethersauerstoff enthaltendes $C_2$-$C_9$-Alkyl, $C_7$-$C_{12}$-Aralkyl, gegebenenfalls substituiertes Aryl, einen Heterocyclus oder einen Heterocyclus enthaltendes $C_1$-$C_{22}$-Alkyl darstellen oder $R^6$ und $R^7$ gemeinsam mit dem oder den Atom(en), an das (die) sie gebunden sind, ein 3 bis 12-gliedriges Ringsystem bilden,

sowie die Säureadditionssalze und Hydrate dieser Verbindungen.

Einzelne Reste $R^1$ und $R^2$ in Formel (I) sind neben Wasserstoff z.B.: Methyl, Ethyl, Propyl, Butyl, Pentyl, Hexyl, Benzyl, Phenylethyl, Phenylpropyl, Phenylbutyl, Methylbenzyl, Phenyl, Tolyl, Carbomethoxy, Carboethoxy, Carbopropoxy oder Carbobutoxy.

Bevorzugt sind für $R^1$ und $R^2$ Methyl, Ethyl, Benzyl, Phenyl und insbesondere Wasserstoff.

X und Y sind unabhängig voneinander insbesondere Sauerstoff, daneben auch Schwefel oder $NR^8$, worin $R^8$ die vorstehenden Bedeutungen besitzt. Alkylreste, beispielsweise $R^3$ und $R^4$, können sowohl unverzweigt als auch verzweigt sein.

Alkylreste in den Verbindungen der allgemeinen Formeln (I) und (Ib) sind beispielsweise $C_1$-$C_{22}$-Alkylreste, insbesondere $C_1$-$C_8$-Alkylreste, besonders bevorzugt $C_1$-$C_4$-Alkylreste, z.B. Methyl, Ethyl, Propyl, Butyl, Pentyl, Hexyl, Heptyl und Octyl.

Beispiele für solche Reste sind

$(CH_2)_n-CH_3$, 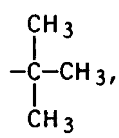, 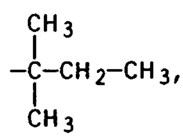, 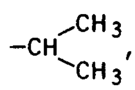, 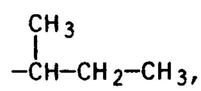, 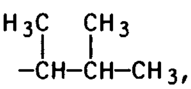,

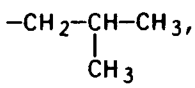, 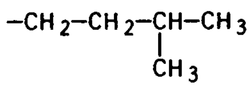 oder 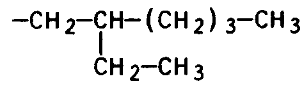 ,

wobei n = 0 - 21 ist.

Ein durch Ethersauerstoff oder Stickstoff unterbrochener Alkylrest für $R^3$ oder $R^4$ oder für $R^6$ und $R^7$ kann beispielsweise sein:

$CH_3-O-CH_2CH_2-$, $CH_3CH_2-O-CH_2CH_2-$, $CH_3-O-CH_2CH_2CH_2-$, $CH_3CH_2-O-CH_2CH_2CH_2-$,

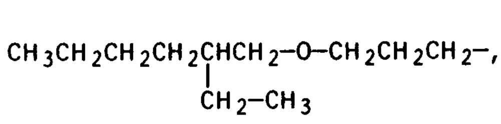, 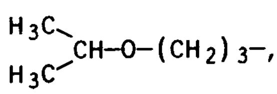, 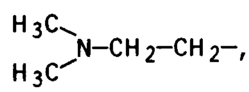,

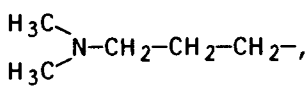, 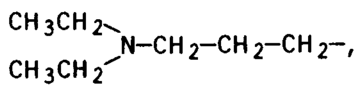, 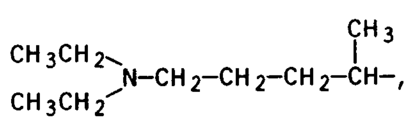,

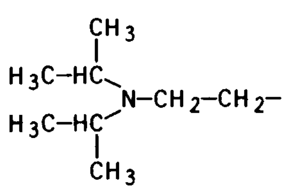 , 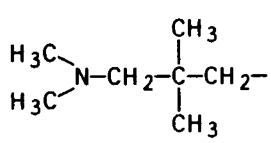 oder 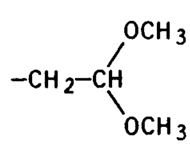 .

Beispielhafte Harnstoffgruppierungen sind die folgenden:

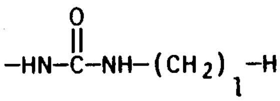 , 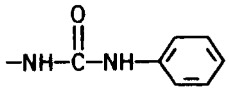 , 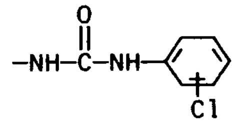 ,

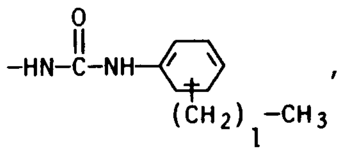 , 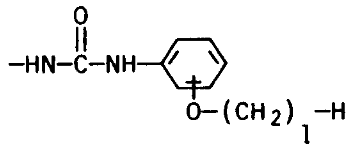 ,

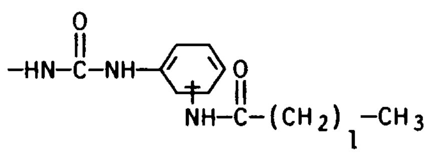 , 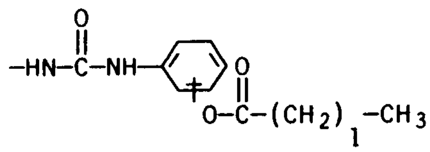 ,

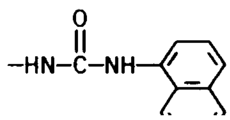 , 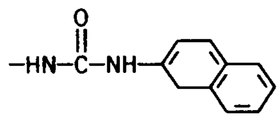 , worin l = 0 bis 22 ist.

Beispielhafte Urethangruppierungen bzw. Carbamoylgruppen sind die folgenden:

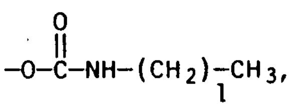 , 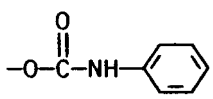 , 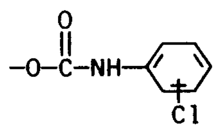 , 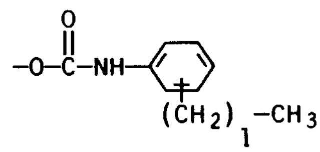

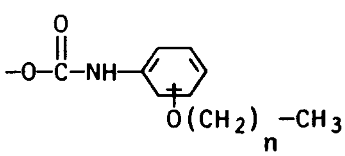 , 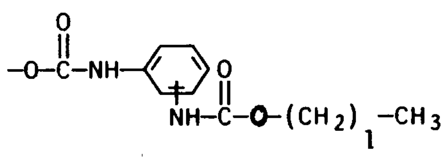 ,

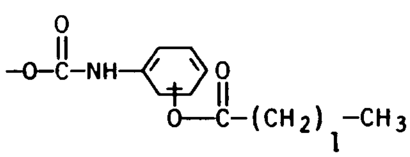 , 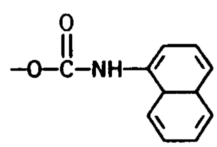 ,

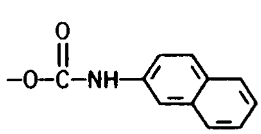 (l = 0 bis 22), 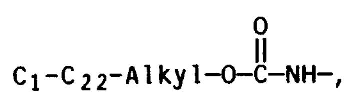 ,

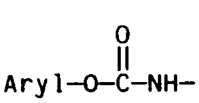 , 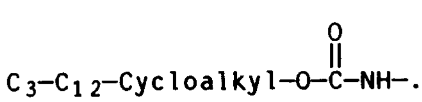 .

Cycloalkylreste (einschließlich Bicycloalkyl) für $R^3$, $R^4$, $R^5$ können beispielsweise sein:

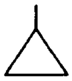 , 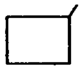 , 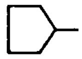 , 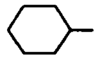 , 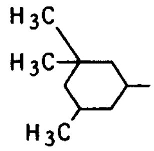 , 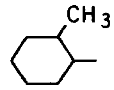 , 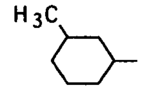 ,

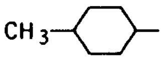 , 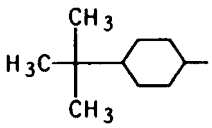 , 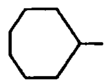 , 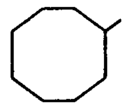 , 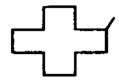 ,

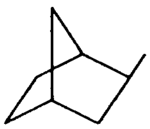 , 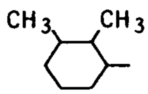 , 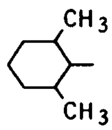 , 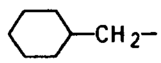 ,

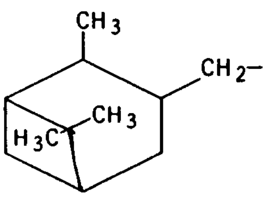 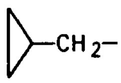 , 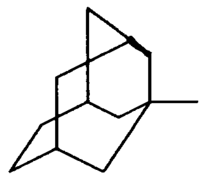

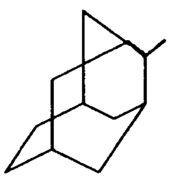 , 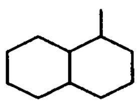 , 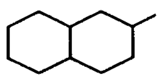 , 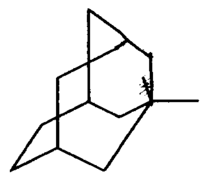

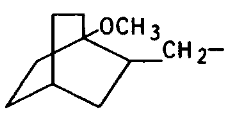 , 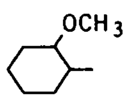 , 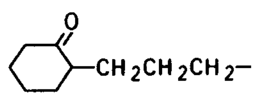 ,

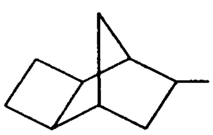 , 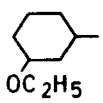 , 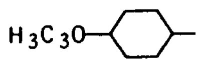 ,

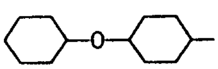 , 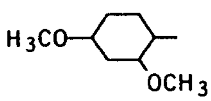 , 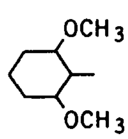 ,

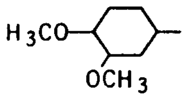, 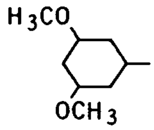, 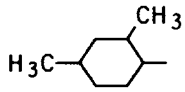, 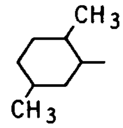

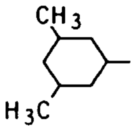, 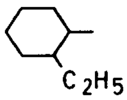, 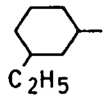, 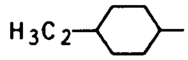, 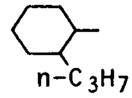,

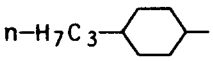, 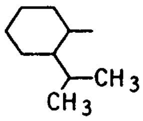, 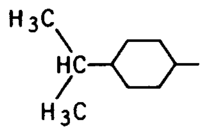, 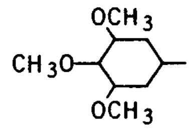,

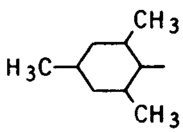, 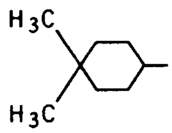, 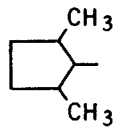,

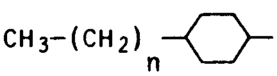, 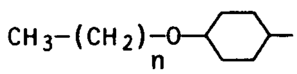, 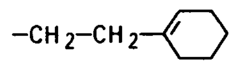.

(n = 1 bis 20)

Aralkylreste $R^3$, $R^4$, $R^5$, $R^6$, $R^7$ können beispielsweise sein:

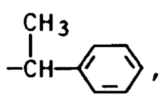, 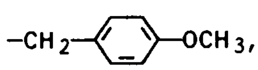, 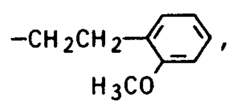,

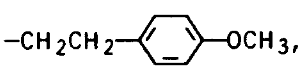, 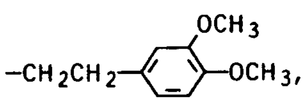, 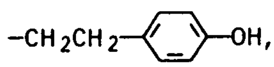, 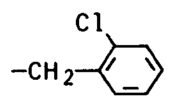,

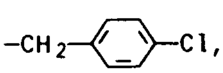, 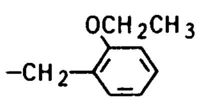,

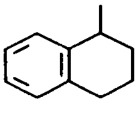, 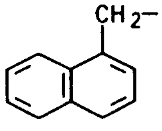, 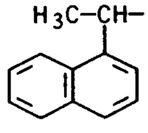, 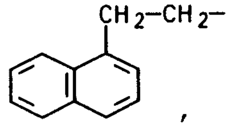,

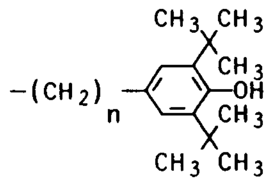 , 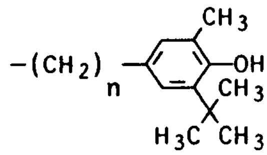 , 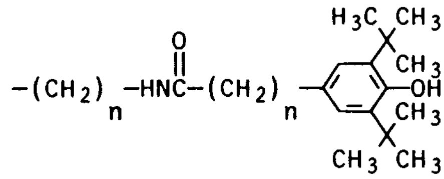 ,

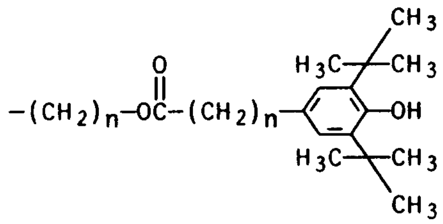 , 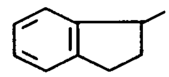 , 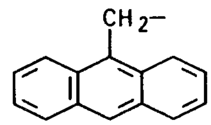 ,

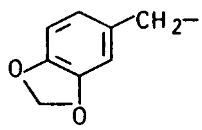 , 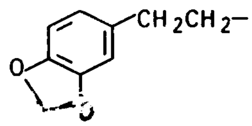 , 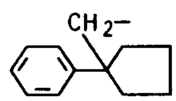 ,

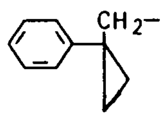 , 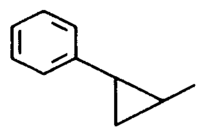 , 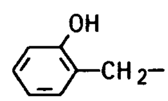 ,

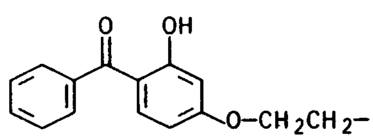 , 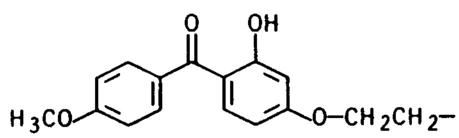 ,

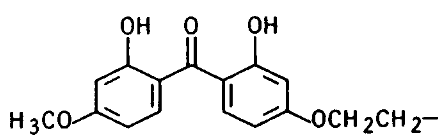 , 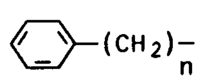 (n = 1 bis 20).

Alkylreste $R^3$, $R^4$, $R^5$, $R^6$, $R^7$, die Heterocyclen enthalten, können beispielsweise sein:

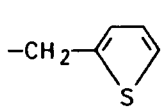 , 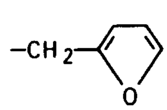 , 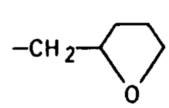 , 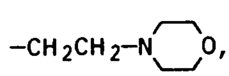 , 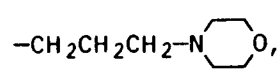 ,

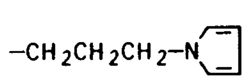 , 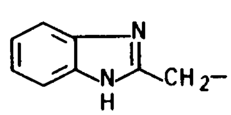 , 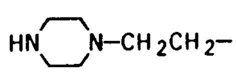 , 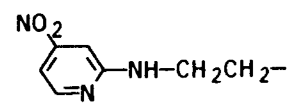 ,

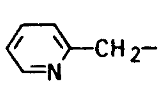 , 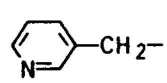 , 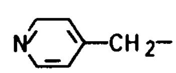 ,

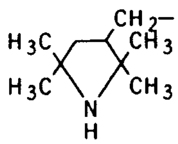 , 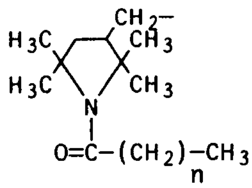 , 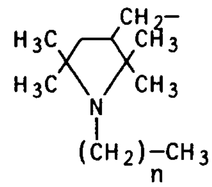 ,

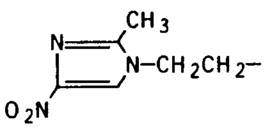 , 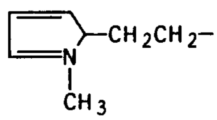 , 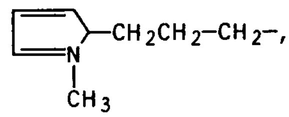,

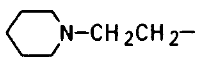 , 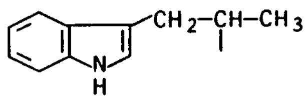 , 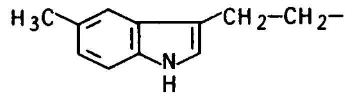 ,

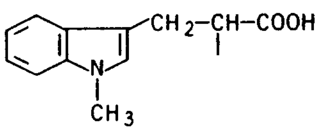 , 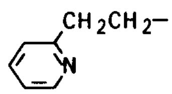 , 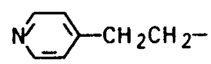 ,

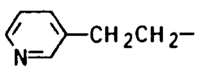 , 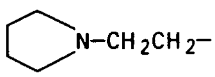 , 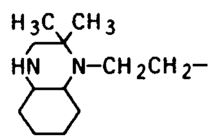 , 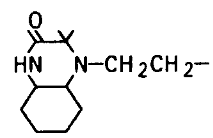 ,

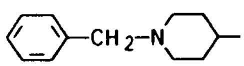 , 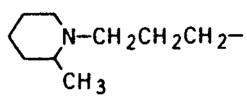 ,

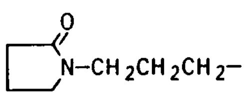 , 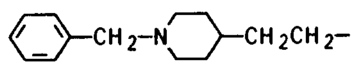 , 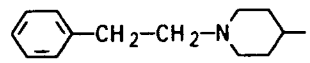 ,

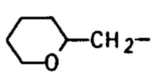 , 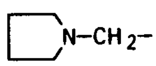 , 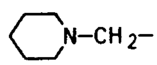 ,

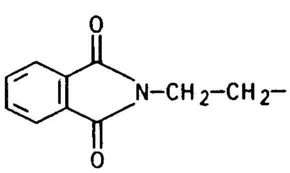 , 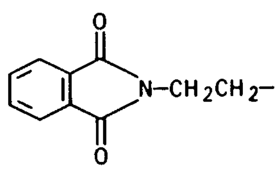 , 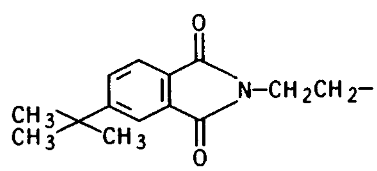 ,

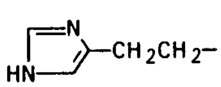 , 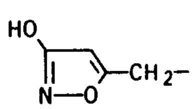 , 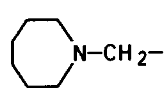 , 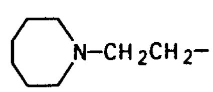 ,

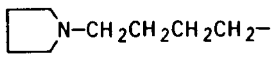, 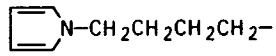, 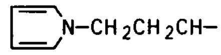,

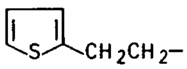, 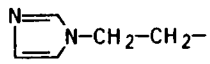, 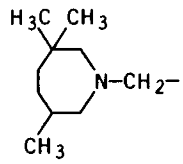, 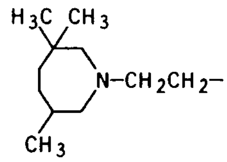,

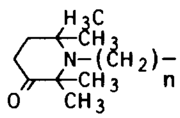, 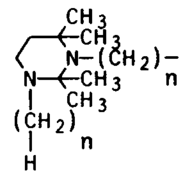, 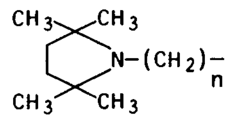,

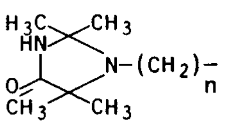, (n = 1 bis 20),

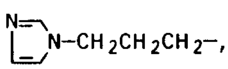, 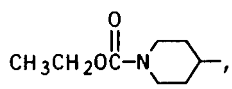, 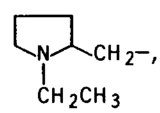

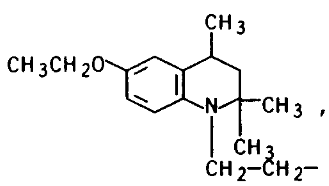, 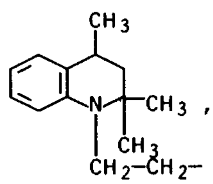, 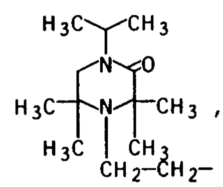,

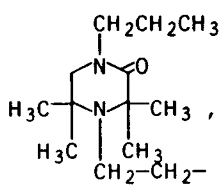, 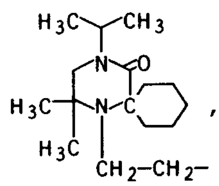, 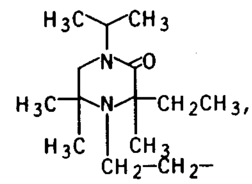

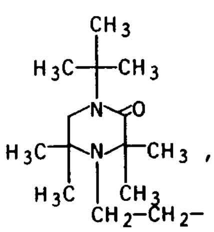, 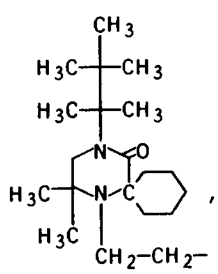, 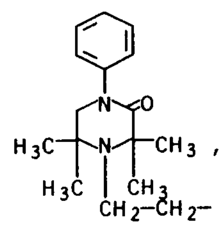, 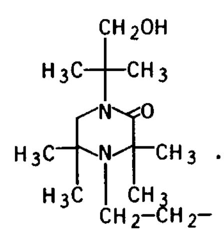.

Alkylreste $R^3$, $R^4$, die Hydroxyl-, Thiol-, Carboxyl- oder Cyangruppen enthalten, können beispielsweise sein:

$-CH_2CH_2-OH$, $-CH_2CH_2CH_2-OH$, 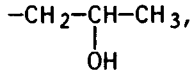, 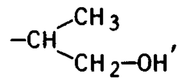, 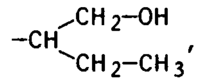,

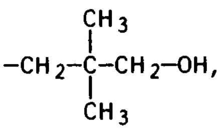, $-CH_2CH_2-SH$, $-(CH_2)_n-COOH$, 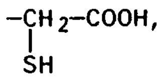, 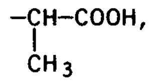, 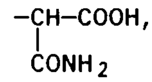,

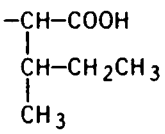, 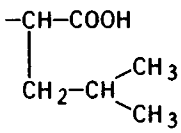, 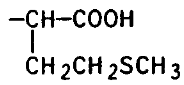, 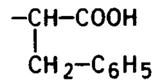, 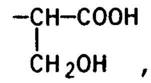,

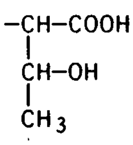, 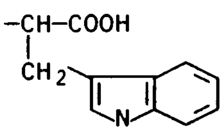, 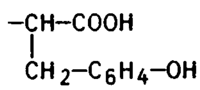, 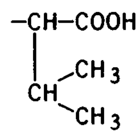, 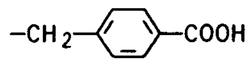,

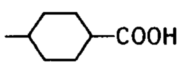, $-(CH_2)_n-CN$ oder 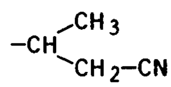, wobei n = 1 bis 20 ist.

Soweit in den Verbindungen (I) bzw. (Ib) nichtaromatische Heterocyclen vorliegen, können diese beispielsweise die folgenden sein:

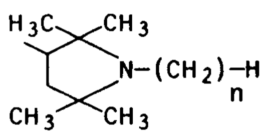, 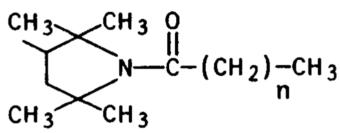, 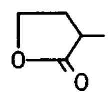,

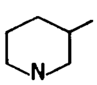, 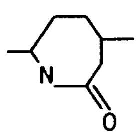, 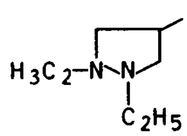, 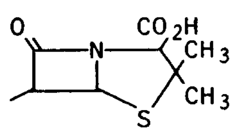,

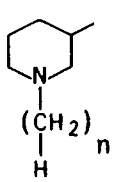, 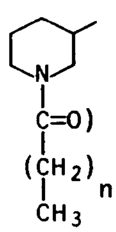, 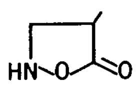, 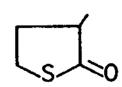,

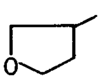, 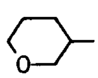, 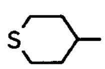, 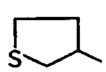,

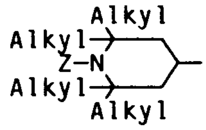 (Z = Wasserstoff, Alkyl, C-Acyl, HO- mit der Maßgabe, daß $R^3 \neq R^4$).

Soweit in den Verbindungen (I) bzw. (Ib) Hydroxy-substituierte oder Carboxyl-substituierte Alkylreste vorliegen, können diese beispielsweise die folgenden sein: Zucker- oder Aminozuckerreste,

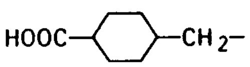 , $HOOC-(CH_2)_n-$ , 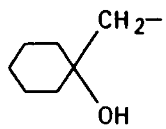 , 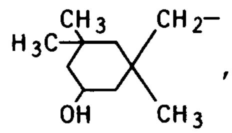 ,

$HO-CH_2CH_2O-CH_2CH_2-$ , $HO-(CH_2)_n-$ (n = 1 bis 20), $H-(CH_2)_n-\overset{O}{\overset{\|}{C}}NH-(CH_2)_m-$ ,

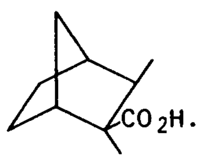 mit n = 1 bis 20.

Brückenglieder -A- sind zweiwertige aliphatische, araliphatische oder aromatische Gruppen, die auch Heteroatome, nämlich Sauerstoff, Stickstoff oder Schwefel, enthalten können.

Insbesondere sind Alkylen-, Cycloalkylen-, Aralkylen-, durch CO oder $SO_2$ substituierte Alkylen-, Aralkylen- oder Arylreste zu nennen.

Einzelne Brückenglieder sind beispielsweise:

$-(CH_2)_p-$, $-(CH_2)_pCH{=}CH-$, $-(CH_2)_p-C{\equiv}C-$, 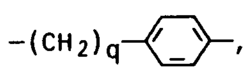, 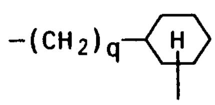,

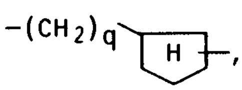, 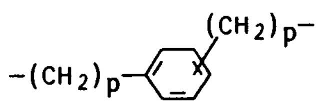 , $-(CH_2)_2O-$, $-(CH_2)_2O(CH_2)_2-$,

$-(CH_2)_3O(CH_2)_2-$, $-\underset{CH_3}{CH}-CH_2-O(CH_2)_2-$, $-(CH_2)_2O\underset{CH_3}{CH}-CH_2-$, $-\underset{CH_3}{CH}-CH_2O\underset{CH_3}{CH}-CH_2-$,

$-\underset{O}{\overset{}{\underset{\|}{C}}}-(CH_2)_q-$, $-CH_2-\underset{C_2H_5}{CH}-(CH_2)_4-$, $-\underset{CH_3}{CH}-CH_2$, $-CH_2-CH{=}CH-$, $-\underset{CH_3}{CH}-CH_2-$, $-\overset{CH_3}{\underset{CH_3}{C}}-CH_2-$,

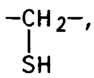 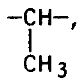 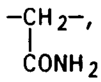 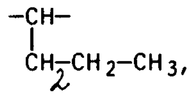 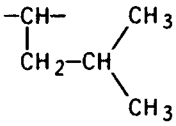 , 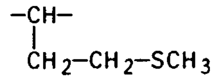

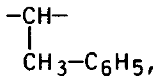 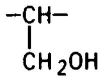 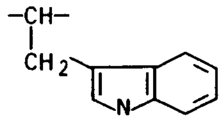 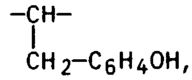 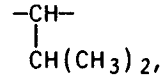

wobei p = 1 bis 20 und q = 0 bis 4 sind.

Bevorzugte Brückenglieder sind $-(CH_2)-_n$, worin n = 1 bis 5, insbesondere 1, 2 und 5 ist.
Weitere Beispiele für Brückenglieder -B- sind die folgenden:

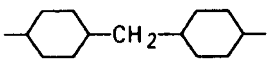 , 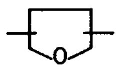 , 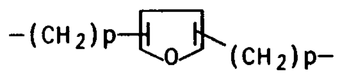 ,

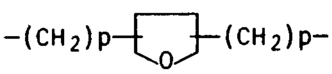 , 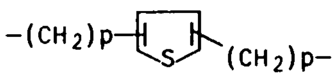 , 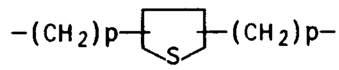 ,

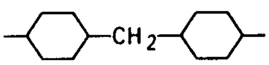 ,

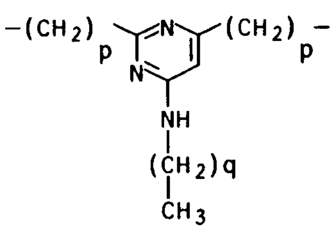 , 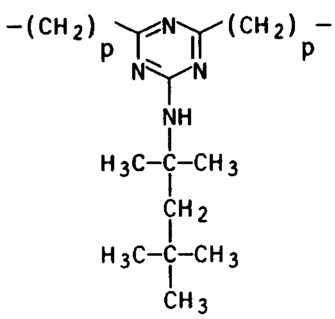 , 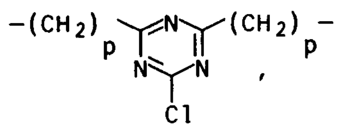 ,

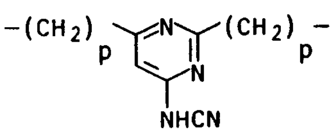 , 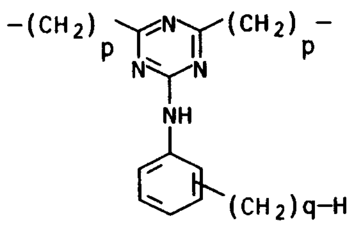 ,

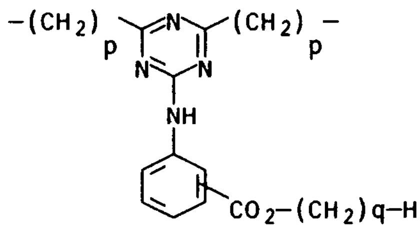 , 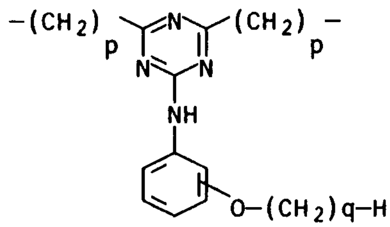 ,

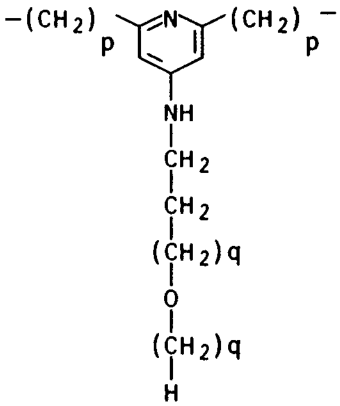

$-(-CH_2CH_2-O-)_p-$ , 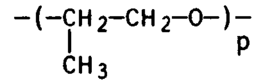 ,

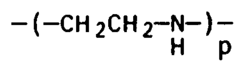 , 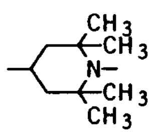 ,

wobei in den vorstehenden Formeln p für 1 bis 20 steht.

Der in Verbindung mit den Restedefinitionen gebrauchte Begriff "gegebenenfalls substituiert" umfaßt die vorstehend erläuterten Bedeutungsmöglichkeiten. Er umfaßt insbesondere Substituenten, wie $C_1$-$C_6$-Alkyl, $C_1$-$C_6$-Alkoxy, $C_2$-$C_{12}$-Carbonester, $C_1$-$C_{12}$-C-Acyl, Halogen, Nitro, Carboxyl, Amino, Hydroxy, -SH, -S-$C_1$-$C_4$-Alkyl, Phenyl, $C_2$-$C_6$-Alkenyl, (Poly)ethoxy, (Poly)amino.

Der Begriff "Acyl" steht beispielsweise für $C_1$-$C_{20}$-Acyl, insbesondere für Reste der Formel

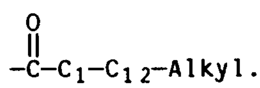

Die Herstellung der Verbindungen (I) bzw. (Ib) ergibt sich aus der FR-A-2 291 203.

So kann man die Verbindungen der allgemeinen Formeln (I) oder (Ib) durch Umsetzung von primären Aminen $R^3$-$NH_2$, Aminocarbonsäureestern, Aminocarbonsäureamiden, Aminosulfonsäuren, Aminosulfonsäureestern oder Aminosulfonsäureamiden, oder entsprechenden Verbindungen, mit Verbindungen der allgemeinen Formel (II) herstellen.

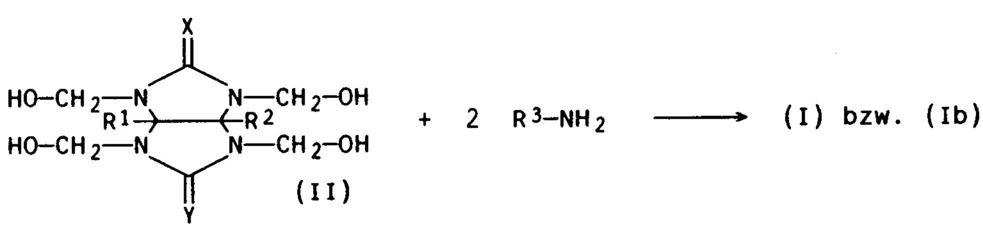


Verbindungen der allgemeinen Formeln (I) oder (Ib) mit $R^3$ ungleich $R^4$ können durch Umsetzung von Verbindungen der allgemeinen Formel (II) mit verschiedenen primären Aminen hergestellt werden. Die verschiedenen primären Amine können dabei gleichzeitig oder nacheinander der Reaktionsmischung zugesetzt werden. In der Regel entstehen dabei Gemische der denkbaren Reaktionsprodukte, die wenn es gewünscht wird durch an sich bekannte Verfahren aufgetrennt werden können.

Verbindungen der allgemeinen Formeln (I) oder (Ib) mit $R^3$ ungleich $R^4$ können weiterhin durch Reaktion von Verbindungen der allgemeinen Formel (IV) mit primären Aminen $R^4$-$NH_2$ und Formaldehyd hergestellt werden.

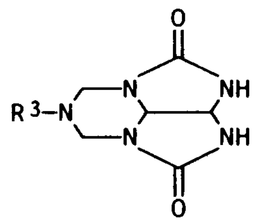

(IV)

Die Verbindungen der allgemeinen Formel (II) können dabei auch in situ durch Reaktion von Verbindungen der allgemeinen Formel (III) mit Formaldehyd oder einer Formaldehydquelle hergestellt werden.

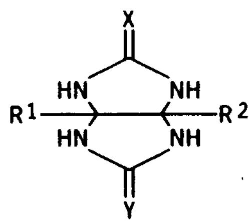

(III).

Die erfindungsgemäßen Verbindungen (Ib) besitzen außerordentlich gute stabilisierende Eigenschaften, haben keine Eigenfarbe, sind gut verträglich mit organischen Polymeren und zeigen einen niedrigen Dampfdruck. Sie sind darüber hinaus auch wertvolle Zwischenprodukte für die Herstellung von Stabilisatoren, Pflanzenschutzmitteln, Pharmaka und Polymeren.

Verbindungen der allgemeinen Formeln (I) und (Ib), worin B- für $-CO_2R^5$ ($R^5$ = $C_1$-$C_{22}$-Alkyl) steht, können beispielsweise durch Aminolyse in Verbindungen mit

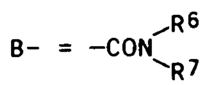

überführt werden.

Die erfindungsgemäß zu verwendenden Verbindungen können in Form der freien Basen oder als Salze vorliegen. Geeignete Anionen stammen z.B. von anorganischen Säuren und insbesondere organischen Carbonsäuren sowie organischen Sulfonsäuren.

Anorganische Anionen sind z.B.: Chlorid, Bromid, Sulfat, Methosulfat, Tetrafluoroborat, Phosphat oder Rhodanid.

Carbonsäure-Anionen sind beispielsweise: Formiat, Acetat, Propionat, Hexanonat, Cyclohexanonat, Lactat, Stearat, Dodecylbenzoat, Benzoat, Acrylat, Methacrylat, Zitrat, Malonat oder Succinat sowie Anionen von Polycarbonsäuren mit bis zu 3000 COOH-Gruppen.

Sulfonsäure-Anionen sind beispielsweise Benzolsulfonat oder Tosylat.

Die erfindungsgemäß zu verwendenden Verbindungen eignen sich zum Stabilisieren von Kunststoffen und Lacken gegen den Abbau durch Licht und Wärme. Sie werden den zu stabilisierenden Kunststoffen in einer Konzentration von 0,01 bis 5 Gew.%, vorzugsweise 0,02 bis 1 Gew.% vor, während oder nach der Polymerbildung zugesetzt.

Zur Vermischung der erfindungsgemäß zu verwendenden Verbindungen mit den zu stabilisierenden Kunststoffen können alle bekannten Vorrichtungen und Methoden zum Einmischen von Stabilisierungsmitteln oder anderen Zusätzen in Polymere angewandt werden.

Die erfindungsgemäß stabilisierten Kunststoffe können gegebenenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die den Kunststoffen neben den erfindungsgemäß zu verwendenden Verbindungen zugesetzt werden können, sind z.B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-$\beta$-(3,5-di-tert.-butly-4-hydro-xyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[β-(3,5-di-tert.-butyl-4-hydroxyphenyl)-propionyloxyethyl]-isocyanurat, 1,3,5-Tris-(2,6,di-methyl-3-hydroxy-4-tert.-butylbenzyl)-isocyanurat, Pentaerythrit-tetrakis-[β-(3,5-di-tert.-butyl-4-hydroxy-phenyl)-propionat], erwähnt.

Als phosphorhaltige Antioxidantien seien beispielsweise Tris-(nonyl-phenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit, Tetrakis-(2,4-di-tert.-butylphenyl)-4,4'-biphenylendiphosphit, erwähnt.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat), Pentaerythrittetrakis-(β-hexylthiopropionat), erwähnt.

Weitere Antioxidantien und Lichtstabilisatoren, die zusammen mit den erfindungsgemäßen Verbindungen verwendet werden können, sind z.B. 2-(2'-Hydroxyphenyl)-benztriazole, 2-Hydroxybenzophenone, Arylester von Hydroxybenzoesäuren, α-Cyanozimtsäurederivate, Nickelverbindungen, Oxalsäuredianilide, Derivate des Vitamin E (Tocopherol) und/oder Benzimidazol-2-carbonsäureanilide.

Als organische Polymere, die erfindungsgemäß stabilisiert werden können, seine beispielsweise genannt:

Polymere von Mono- und Diolefinen, wie z.B. Polyethylen niedriger oder hoher Dichte, lineares Polyethylen niedriger Dichte, Polypropylen, Polyisobutylen, Polybuten-1, Polyisopren, Polybutadien sowie Copolymerisate von Mono- oder Diolefinen oder Mischungen der genannten Polymeren;

Copolymerisate von Mono- oder Diolefinen mit anderen Vinylmonomeren wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polystyrol;

Copolymere von Styrol oder α-Methylstyrol mit Dienen oder Acrylderivaten, wie z.B. Styrol-Butadien, Styrol-Acrylnitril, Styrol-Ethylmethacrylat, Styrol-Butadien-Ethylacrylat, Styrol-Acrylnitril-Methacrylat;

ABS-, MBS- oder ähnliche Polymere;

Halogenhaltige Polymere, wie z.B. Polyvinylchlorid, Polyvinylfluorid, Polyvinylidenfluorid sowie dere Copolymere;

Polymere, die sich von α,β-ungesättigten Säuren und deren Derivaten ableiten, wie Polyacrylate und Polymethacrylate, Polyacrylamide und Polyacrylnitrile;

Polymere, die sich von ungesättigten Alkoholen und Aminen bzw. deren Acrylderivaten oder Acetalen ableiten, wie Polyvinylalkohol oder Polyvinylacetat;

Polyurethane, Polyamide, Polyharnstoffe, Polyester, Polycarbonate, Polysulfone, Polyethersulfone und Polyetherketone.

Weitere organische Polymere, die erfindungsgemäß stabilisiert werden können, sind Industrielackierungen. Unter diesen sind Einbrennlackierungen, unter diesen wiederum Fahrzeuglackierungen, vorzugsweise Zweischichtlackierungen, besonders hervorzuheben.

Auch hier können zusätzlich die bereits aufgeführten Antioxidantien und Lichtschutzmittel verwendet werden.

Die erfindungsgemäß zu verwendenden Verbindungen können in fester oder gelöster Form dem Lack zugesetzt werden. Ihre gute Löslichkeit in Lacksystemen ist dabei von besonderem Vorteil.

Bevorzugt ist die Verwendung in Polyolefinen, vorzugsweise Ethylen- und Propylenpolymerisaten.

Weiterhin bevorzugt ist die Verwendung in Polyurethanen, vorzugsweise in Kombination mit einem UV-Absorber und/oder einem Antioxidans.

Die Erfindung wird durch die nachstehenden Beispiele weiter erläutert.

Allgemeine Herstellvorschrift für Verbindungen der allgemeinen Formel (I) mit $R^1$ = $R^2$ = Wasserstoff, X = Y = Sauerstoff.

Zu 400 ml Wasser werden 262 g (= 0,5 Mol) einer 50 %igen wäßrigen Lösung von Tetramethylolacetylendiharnstoff gegeben. Anschließend werden 1,0 Mol primäres Amin $R^3$-$NH_2$ zugesetzt. Die Mischung wird 2,5 h auf 80 - 100 °C erhitzt. Die Aufarbeitung geschieht nach 2 Methoden.

A) Fällt das Produkt kristallin an, wird abgesaugt und im Wasserstrahlvakuum getrocknet. Eine weitere Reinigung entfällt in der Regel.

B) Fällt das Produkt ölig an, wird die wäßrige Phase abdekantiert und die ölige Phase zur Entfernung letzter Wasserspuren im Wasserstrahlvakuum eingeengt. Der Rückstand wird umkristallisiert.

Die nach dieser Herstellvorschrift hergestellten Verbindungen sind in Tabelle 1 zusammengefaßt.

Tabelle 1: Herstellbeispiele für Verbindungen der allgemeinen Formel (I) mit $R^1$ = $R^2$ = Wasserstoff und X = Y = Sauerstoff

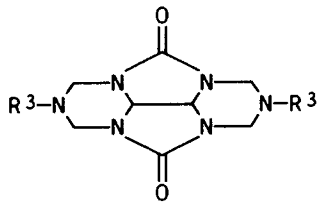

| Bsp. | $R^3$ | Schmp. [°C] | Analyse | C [%] | H [%] | N [%] | O [%] |
|---|---|---|---|---|---|---|---|
| 1 | $-CH_3$ | 285 | Ber.<br>Gef. | 47,6<br>47,3 | 6,4<br>6,4 | 33,3<br>33,2 | 12,7<br>13,6 |
| 2 | $-CH_2CH_3$ | 210-212 | Ber.<br>Gef. | 51,4<br>51,3 | 7,2<br>7,2 | 30,0<br>29,9 | 11,5<br>11,5 |
| 3 | $-CH_2CH_2CH_3$ | 110-112 | Ber.<br>Gef. | 54,5<br>54,5 | 7,8<br>7,9 | 27,3<br>27,0 | 10,4<br>10,5 |
| 4 | $-CH_2CH_2CH_2CH_3$ | 94-95 | Ber.<br>Gef. | 57,1<br>57,1 | 8,4<br>8,6 | 25,0<br>25,0 | 9,5<br>9,5 |
| 5 | 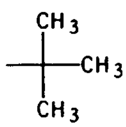 | 231 | Ber.<br>Gef. | 57,1<br>57,1 | 8,4<br>8,4 | 25,0<br>25,0 | 9,5<br>9,5 |
| 6 | 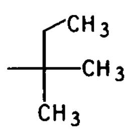 | 119 | Ber.<br>Gef. | 59,3<br>59,2 | 8,8<br>8,9 | 23,1<br>22,8 | 8,8<br>9,2 |
| 7 | $-CH_2-CH=CH_2$ | 90-92 | Ber.<br>Gef. | 55,2<br>54,9 | 6,6<br>6,7 | 27,6<br>27,3 | 10,5<br>10,9 |
| 8 | 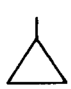 | 242-245 | Ber.<br>Gef. | 55,3<br>55,5 | 6,6<br>6,7 | 27,6<br>27,6 | 10,5<br>10,5 |
| 9 | 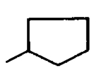 | 212-213 | Ber.<br>Gef. | 60,0<br>60,2 | 7,8<br>8,0 | 23,3<br>23,1 | 8,9<br>9,0 |

Tabelle 1: (Forts.)

| Bsp. | $R^3$ | Schmp. [°C] | | Analyse C [%] | H [%] | N [%] | O [%] |
|---|---|---|---|---|---|---|---|
| 10 | 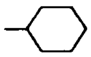 | 228 | Ber.<br>Gef. | 61,8<br>61,8 | 8,3<br>8,3 | 21,6<br>21,5 | 8,2<br>8,5 |
| 11 | 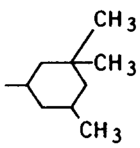 | 217-220 | Ber.<br>Gef. | 66,1<br>66,0 | 9,4<br>9,3 | 17,8<br>17,8 | 6,8<br>6,8 |
| 12 | $-CH_2CH_2OCH_3$ | 118-120 | Ber.<br>Gef. | 49,4<br>49,5 | 7,1<br>7,1 | 24,7<br>24,9 | 18,8<br>19,0 |
| 13 | $-CH_2CH_2OCH_2CH_3$ | 67-69 | Ber.<br>Gef. | 52,2<br>51,6 | 7,7<br>7,7 | 22,8<br>22,5 | 17,4<br>17,8 |
| 14 | $-CH_2CH_2-OH$ | 241 | Ber.<br>Gef. | 46,2<br>46,2 | 6,5<br>6,6 | 26,9<br>26,8 | 20,5<br>20,6 |
| 15 | $-CH_2CH_2CH_2OH$ | 124-126 | Ber.<br>Gef. | 49,4<br>49,3 | 7,2<br>7,2 | 24,7<br>24,5 | 18,8<br>19,3 |
| 16 | 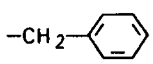 | 229-231 | Ber.<br>Gef. | 65,3<br>65,1 | 6,0<br>6,1 | 20,8<br>20,5 | 7,9<br>8,1 |
| 17 | 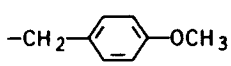 | 156-158 | Ber.<br>Gef. | 62,1<br>61,8 | 6,1<br>6,1 | 18,1<br>18,0 | 13,8<br>13,9 |
| 18 | 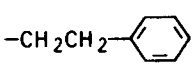 | 152-154 | Ber.<br>Gef. | 66,6<br>66,6 | 6,5<br>6,7 | 19,4<br>19,4 | 7,4<br>7,3 |
| 19 | 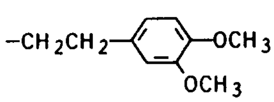 | 166-168 | Ber.<br>Gef. | 60,8<br>60,5 | 6,6<br>6,7 | 15,2<br>15,4 | 17,4<br>17,7 |
| 20 | 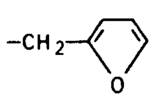 | 147-149 | Ber.<br>Gef. | 56,2<br>56,2 | 5,2<br>5,4 | 21,9<br>21,9 | 16,6<br>16,8 |

Tabelle 1: (Forts.)

| Bsp. | $R^3$ | Schmp. [°C] | | Analyse C [%] | H [%] | N [%] | O [%] |
|---|---|---|---|---|---|---|---|
| 21 | 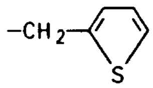 | 213-216 | Ber.<br>Gef. | 51,9<br>52,2 | 4,8<br>5,0 | 20,2<br>20,0 | 7,7<br>7,7 |
| 22 | 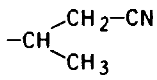 | 206-207 | Ber.<br>Gef. | 53,6<br>53,6 | 6,2<br>6,3 | 31,3<br>31,1 | 8,9<br>9,0 |
| 23 | 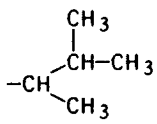 | 149-151 | Ber.<br>Gef. | 59,3<br>59,3 | 8,8<br>8,9 | 23,1<br>23,1 | 8,8<br>8,9 |
| 24 | 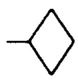 | 261-264 | Ber.<br>Gef. | 57,8<br>57,9 | 7,3<br>7,4 | 25,3<br>25,4 | 9,6<br>9,8 |
| 25 | 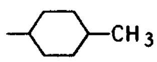 | 136-139 | Ber.<br>Gef. | 63,4<br>63,3 | 8,7<br>8,7 | 20,2<br>19,8 | 7,7<br>7,8 |
| 26 | 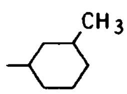 | 133-137 | Ber.<br>Gef. | 63,4<br>63,2 | 8,7<br>8,7 | 20,2<br>19,9 | 7,7<br>7,7 |
| 27 | 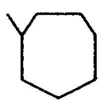 | 165-167 | Ber.<br>Gef. | 63,4<br>63,0 | 8,7<br>8,7 | 20,2<br>20,0 | 7,7<br>7,8 |
| 28 | 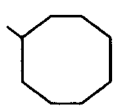 | 170-171 | Ber.<br>Gef. | 64,8<br>64,5 | 9,1<br>9,1 | 18,9<br>18,6 | 7,2<br>7,3 |
| 29 | $-(CH_2)_4-CH_3$ | 86-89 | Ber.<br>Gef. | 59,3<br>59,0 | 8,9<br>8,9 | 23,1<br>22,7 | 8,8<br>9,0 |

Tabelle 1: (Forts.)

| Bsp. | $R^3$ | Schmp. [°C] | | Analyse C [%] | H [%] | N [%] | O [%] |
|---|---|---|---|---|---|---|---|
| 30 | 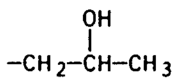 | 189-191 | Ber.<br>Gef. | 49,4<br>49,2 | 7,1<br>7,2 | 24,7<br>24,7 | 18,8<br>18,9 |
| 31 | 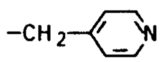 | 218-220 | Ber.<br>Gef. | 59,1<br>58,9 | 5,5<br>5,6 | 27,6<br>27,4 | 7,9<br>8,1 |
| 32 | 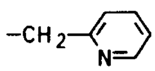 | 202-204 | Ber.<br>Gef. | 59,1<br>58,9 | 5,5<br>5,6 | 27,6<br>27,4 | 7,9<br>8,0 |
| 33 | 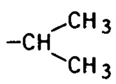 | 168-170 | Ber.<br>Gef. | 54,5<br>54,6 | 7,8<br>7,8 | 27,3<br>27,2 | 10,4<br>10,4 |
| 34 | 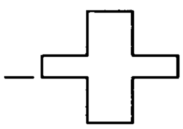 | 188-190 | Ber.<br>Gef. | 69,0<br>68,8 | 10,1<br>10,2 | 15,1<br>15,1 | 5,7<br>5,9 |
| 35 | $-(CH_2)_{17}-CH_3$ | 70-72 | Ber.<br>Gef. | 72,5<br>72,7 | 11,6<br>11,7 | 11,5<br>11,4 | 4,4<br>4,6 |
| 36 | 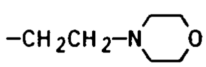 | 125-127 | Ber.<br>Gef. | 53,4<br>53,4 | 7,6<br>7,7 | 24,9<br>24,7 | 14,2<br>14,1 |
| 37 | 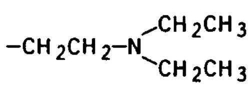 | 96-99 | Ber.<br>Gef. | 56,8<br>56,4 | 9,1<br>9,2 | 26,5<br>26,3 | 7,6<br>8,4 |
| 38 | 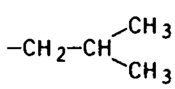 | 123-125 | Ber.<br>Gef. | 57,1<br>57,1 | 8,4<br>8,5 | 25,0<br>24,9 | 9,5<br>9,5 |
| 39 | 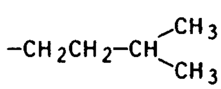 | 98 | Ber.<br>Gef. | 59,3<br>59,3 | 8,9<br>8,9 | 23,1<br>23,0 | 8,8<br>8,8 |

Tabelle 1: (Forts.)

| Bsp. | $R^3$ | Schmp. [°C] | | Analyse C [%] | H [%] | N [%] | O [%] |
|---|---|---|---|---|---|---|---|
| 40 | $-CH_2CH_2OCH_2CH_2OH$ | 105-108 | Ber.<br>Gef. | 48,0<br>47,6 | 7,0<br>7,2 | 21,0<br>20,7 | 24,0<br>24,5 |
| 41 | $-(CH_2)_5-CN$ | 121-123 | Ber.<br>Gef. | 57,9<br>57,9 | 7,3<br>7,5 | 27,0<br>26,9 | 7,7<br>7,9 |
| 42 | 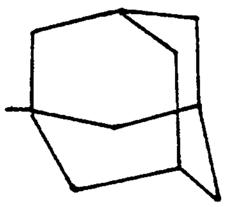 | 277-279 | Ber.<br>Gef. | 68,3<br>68,0 | 8,2<br>8,3 | 17,1<br>16,9 | 6,5<br>6,8 |
| 43 | 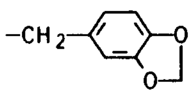 | 280 | Ber.<br>Gef. | 58,5<br>58,5 | 4,9<br>5,0 | 17,1<br>16,7 | 19,5<br>19,7 |
| 44 | 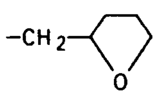 | 95-98 | Ber.<br>Gef. | 55,1<br>54,2 | 7,2<br>7,1 | 21,4<br>21,1 | 16,3<br>17,3 |
| 45 | 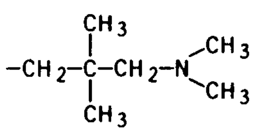 | 140 | Ber.<br>Gef. | 58,6<br>58,6 | 9,4<br>9,5 | 24,9<br>24,8 | 7,1<br>7,1 |
| 46 | 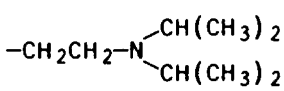 | 168-170 | Ber.<br>Gef. | 61,2<br>60,3 | 8,1<br>9,8 | 23,8<br>23,3 | 6,8<br>6,6 |
| 47 | 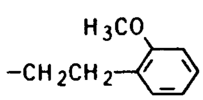 | 140 | Ber.<br>Gef. | 63,4<br>63,4 | 6,5<br>6,7 | 17,1<br>17,0 | 13,0<br>13,1 |
| 48 | 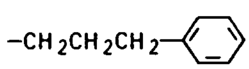 | 126-129 | Ber.<br>Gef. | 67,8<br>67,4 | 7,0<br>7,1 | 18,2<br>18,4 | 6,9<br>7,1 |

Tabelle 1: (Forts.)

| Bsp. | $R^3$ | Schmp. [°C] | | Analyse C [%] | H [%] | N [%] | O [%] |
|---|---|---|---|---|---|---|---|
| 49 | 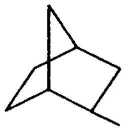 | 210 | Ber.<br>Gef. | 64,1<br>64,0 | 7,8<br>7,9 | 20,4<br>20,3 | 7,8<br>7,9 |
| 50 | 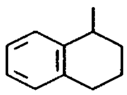 | 212-214 | Ber.<br>Gef. | 69,4<br>69,1 | 6,6<br>6,8 | 17,4<br>17,5 | 6,6<br>6,8 |
| 51 | $-CH_2CH_2CH_2OCH(CH_3)_2$ | 70-71 | Ber.<br>Gef. | 55,3<br>56,2 | 10,6<br>8,2 | 19,4<br>19,3 | 14,5<br>16,0 |
| 52 | $-CH_2CH_2-$ 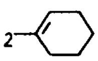 | 98 | Ber.<br>Gef. | 65,4<br>64,4 | 8,2<br>8,3 | 19,1<br>18,8 | 7,3<br>7,8 |
| 53[1)] | $-(CH_2)_{11}-CH_3$ | 58 | Ber.<br>Gef. | 68,5<br>68,5 | 10,8<br>11,0 | 15,0<br>14,7 | 5,7<br>5,8 |
| 54 | $-(CH_2)_5-CH_3$ | 46 | | | | | |
| 55 | $-CH_2CH_2(OCH_3)_2$ | 94 | Ber.<br>Gef. | 48,0<br>47,8 | 7,0<br>7,1 | 21,0<br>20,6 | 24,0<br>24,1 |
| 56 | $-(CH_2)_3-OCH_3$ | 73 | Ber.<br>Gef. | 52,2<br>52,0 | 7,7<br>7,6 | 22,8<br>22,3 | 17,4<br>18,7 |
| 57[1)] | $-(CH_2)_8-CH{=}CH-(CH_2)_7-CH_3$ | Öl | Ber.<br>Gef. | 72,9<br>72,6 | 11,1<br>11,2 | 11,6<br>11,3 | 4,4<br>4,5 |
| 58[1)] | $-(CH_2)_3-$ 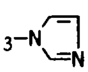 | 135-137 | Ber.<br>Gef. | 54,5<br>54,0 | 6,4<br>6,6 | 31,8<br>30,9 | 7,3<br>8,9 |
| 59[1)] | $-(CH_3)_3-$ 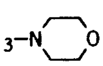 | 140 | Ber.<br>Gef. | 55,2<br>55,1 | 8,0<br>8,0 | 23,4<br>22,9 | 13,4<br>13,5 |

Tabelle 1: (Forts.)

| Bsp. | $R^3$ | Schmp. [°C] | Analyse | C [%] | H [%] | N [%] | O [%] |
|---|---|---|---|---|---|---|---|
| 60 1) | 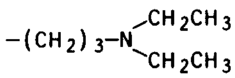 | 73 | Ber.<br>Gef. | 58,6<br>57,5 | 9,4<br>9,5 | 24,9<br>24,1 | 7,1<br>8,7 |
| 61 1) | 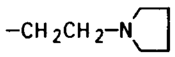 | 134 | Ber.<br>Gef. | 57,4<br>57,2 | 8,2<br>8,2 | 26,8<br>26,6 | 7,7<br>7,7 |
| 62 1) | 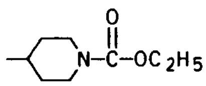 | 175 | Ber.<br>Gef. | 53,9<br>53,6 | 7,2<br>7,2 | 21,0<br>20,7 | 18,0<br>18,0 |
| 63 1) | 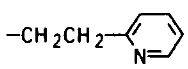 | 142 | Ber.<br>Gef. | 60,8<br>60,5 | 6,0<br>6,2 | 25,8<br>25,6 | 7,6<br>7,6 |
| 64 1) | 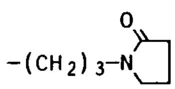 | 168 | Ber.<br>Gef. | 55,7<br>55,6 | 7,2<br>7,4 | 23,6<br>23,7 | 13,5<br>13,8 |
| 65 1) | 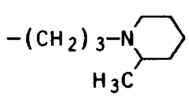 | 85 | Ber.<br>Gef. | 62,1<br>61,2 | 9,2<br>9,2 | 22,3<br>21,7 | 6,4<br>7,4 |
| 66 1) | 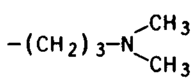 | 72 | Ber.<br>Gef. | 54,8<br>54,4 | 8,7<br>8,8 | 28,4<br>27,6 | 8,1<br>9,5 |
| 67 1) | 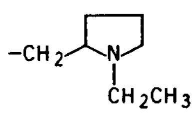 | 95 | Ber.<br>Gef. | 59,2<br>59,3 | 8,6<br>8,6 | 25,1<br>24,9 | 7,2<br>7,3 |
| 68 1) | 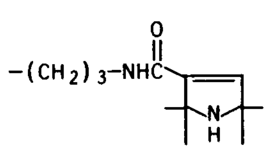 | 118-22 | | | | | |

1) Diese Verbindungen wurden nicht in Wasser hergestellt. Die Ausgangsmaterialien wurden solange in iso-Butanol am Wasserabscheider gekocht, bis kein Wasser mehr abgeschieden wurde. Das Produkt wurde entweder direkt abgesaugt oder nach dem Einengen umkristallisiert.

Beispiel 69

349 g (= 2,50 Mol) Glycinethylesterhydrochlorid werden in 380 ml Wasser gelöst. Unter Eiskühlung gibt man 100 g festes Natriumhydroxid portionsweise zu. Anschließend werden 656 g (= 1,25 Mol) einer 50

%igen wäßrigen Lösung von Tetramethylolacetylendiharnstoff zugegeben. Man hält 2 h am Rückfluß, läßt abkühlen, saugt ab, wäscht mit wenig Wasser nach und trocknet im Wasserstrahlvakuum bei 80 ° C. Man isoliert 199 g (40 %) farblosen Feststoff vom Schmelzpunkt 164 ° C.

Beispiel 70

64 g (= 0,416 Mol) β-Alaninethylesterhydrochlorid in 100 ml Wasser, 33 g 50 %ige Natronlauge und 108,9 g (= 0,208 Mol) 50 %ige wäßrige Lösung von Tetramethylolacetylendiharnstoff werden wie in Beispiel 1 beschrieben umgesetzt. Man isoliert 46 g (52 %) farblosen Feststoff vom Schmelzpunkt 128 ° C.

Beispiel 71

62,5 g (= 0,5 Mol) 2-Aminoethanolsulfonsäure in 100 ml Wasser, 40 g 50 %ige Natronlauge und 131 g (= 0,25 Mol) 50 %ige wäßrige Lösung von Tetramethylolacetylendiharnstoff werden 5 h bei 80 ° C gerührt. Mit Schwefelsäure wird der pH-Wert auf 4,5 eingestellt, die Lösung wird zu zwei Dritteln eingeengt und der ausgefallene Niederschlag abgesaugt. Man kocht diesen mit Ethanol aus, trocknet bei 80 ° C im Wasserstrahlvakuum und isoliert 64 g (58 %) farblosen Farbstoff vom Schmelzpunkt 270 ° C (Zers.).

Beispiel 72

39 g 2,2,6,6-Tetramethyl-4-aminopiperidin, 26,8 g Benzylamin und 131 g einer wäßrigen 50 %igen Lösung von Tetramethylolacetylendiharnstoff wurden in 600 ml iso-Butanol am Wasserabscheider gekocht. Anschließend wurde eingeengt, mit Wasser aufgekocht und getrocknet. Das farblose Produkt hatte einen Zersetzungspunkt von 182 - 184 ° C und war ein Gemisch des Produktes aus Beispiel 16 und der Verbindungen (X) und (XI) im Molverhältnis 1:1:2 ($^1$H-NMR-spektroskopisch).

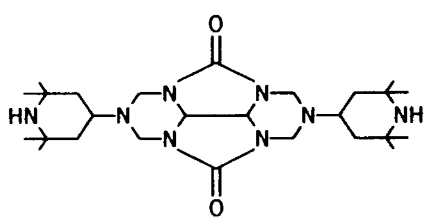

(X)

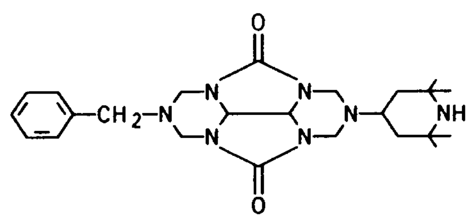

(XI)

Beispiel 73

39 g 2,2,6,6-Tetramethyl-4-aminopiperidin, 64,4 g Octadecylamin und 131 g einer wäßrigen 50 %igen Lösung von Tetramethylolacetylendiharnstoff wurden wie in Beispiel 72 umgesetzt. Man erhielt ein Gemisch der Verbindungen (X), (XI) und (XII) im Molverhältnis 1:1:2 (Schmp. 64 - 65 ° C).

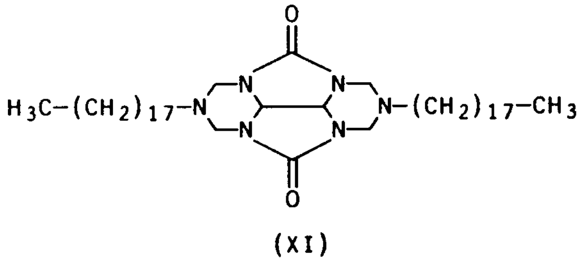

(XI)

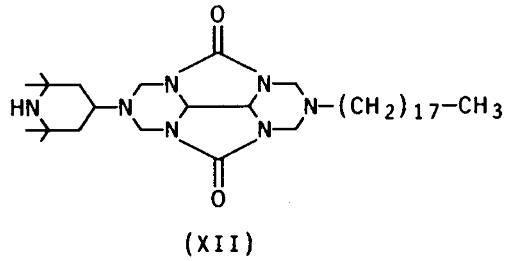

(XII)

Beispiel 74

39 g 2,2,6,6-Tetramethyl-4-aminopiperidin, 24,8 g Cyclohexylamin und 131 g 50 %ige wäßrige Lösung von Tetramethylolacetylendiharnstoff wurden in 500 ml Wasser 3 h gekocht. Der ausgefallene Niederschlag bestand aus dem Produkt aus Beispiel 10 und den Verbindungen (X) und (XIII) im Molverhältnis 1:1:8 (Schmp. 235 -. 237 ° C).

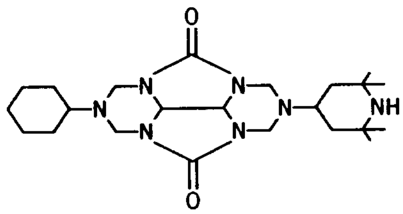

(XIII)

Beispiel 75

17 g 1,5-Dimethyl-2,4,6,8-tetraazabicyclo[3,3,0]octan-3,7-dion, 40 g 30 %ige Formaldehydlösung und 19,8 g Cyclohexylamin wurden in 250 ml iso-Butanol solange am Wasserabscheider gekocht, bis kein Wasser mehr abgeschieden wurde. Man filtrierte, engte ein und digerierte den Rückstand mit Petrolether. Nach dem Absaugen und Trocknen erhielt man die Verbindung der Formel (XIV) als farblosen Feststoff vom Schmp. 173 ° C.

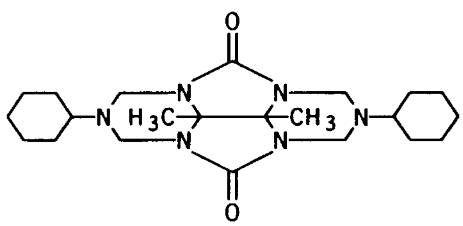

(XIV)

Beispiel 76

Zu 12 g des Produkts aus Beispiel 14 in 100 ml Pyridin tropfte man unter Kühlung 17 g Benzoylchlorid und ließ 1 h bei Raumtemperatur und 1 weitere Stunde bei 60 bis 65 ° C rühren. Man saugte ab, wusch mit Pyridin und Wasser nach. Umkristallisation aus Methanol lieferte die Verbindung der Formel (XV) als farblosen Feststoff vom Schmp. 162 ° C (Zers.).

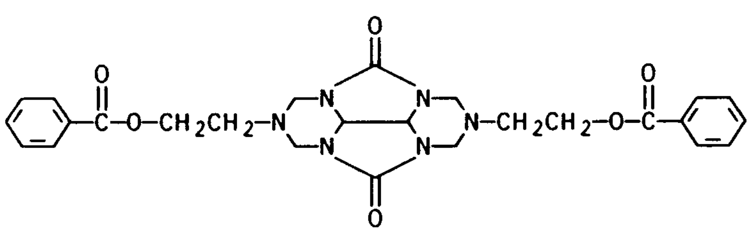

(XV)

Beispiel 77

Zu 20 g des Produkts aus Beispiel 14 in 100 ml Pyridin tropfte man 15,2 g Phenylisocyanat und rührte 4,5 h bei Raumtemperatur. Man goß in Wasser ein, saugte ab, wusch mit Wasser und kristallisierte aus n-Butanol um. Man erhielt die Verbindung der Formel (XVI) als farblosen Feststoff vom Schmp. 220 °C.

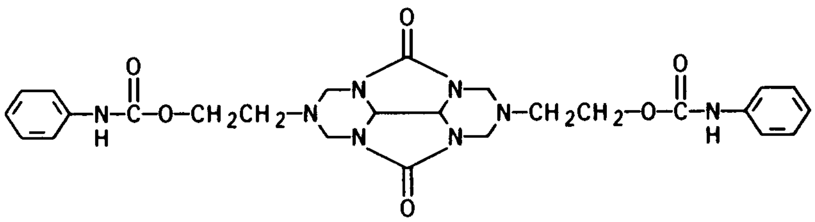

(XVI)

Beispiel 78

31,2 g des Produkts aus Beispiel 14, 100 g 2,6-Di-t-butyl-4-hydroxyphenylpropinsäuremethylester und 5 ml Tetrabutylorthotitanat wurden auf 160 °C geheizt. Man legte Wasserstrahlvakuum an und heizte 6 h lang auf 180 °C. Nach dem Abkühlen wurde mit 300 ml Cyclohexan aufgerührt und abgesaugt. Man erhielt die Verbindung der Formel (XVII) als farblosen Feststoff vom Schmp. 109 bis 111 °C (Zers.).

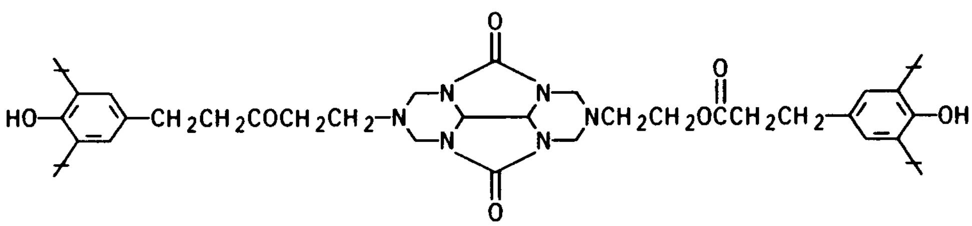

(XVII)

Beispiel 79

6,4 g 2,6-Di-t-butyl-4-aminomethylphenol und 3,5 g Tetramethylolacetylendiharnstoff wurden in 70 ml n-Butanol 3,75 h gekocht. Man filtrierte, engte ein und rührte mit n-Hexan auf. Nach Umkristallisation des Ethanol-Wasser (9:1) erhielt man die Verbindung der Formel (XVIII) als farblosen Feststoff vom Schmp. 260 °C.

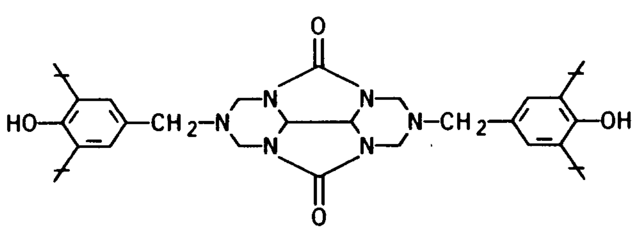

(XVIII)

Beispiel 80

23 ml mit Kaliumcarbonat gesättigtes Ethanol, 27,6 g Paraformaldehyd, 2,2 g Kaliumcarbonat und 78,2 g Acetoncyanhydrin wurden 2 h bei 25 °C gerührt und anschließend mit 85 %iger Phosphorsäure auf pH = 6 eingestellt. Man gab 175 ml Ethanol und 100 g 6-Ethoxy-1,2,3,4-tetrahydro-2,2,4-trimethylchinolin zu und kochte 16,5 h am Rückfluß. Man engte im Wasserstrahlvakuum ein und destillierte im Ölpumpenvakuum. Man erhielt 1-Cyanmethyl-6-ethoxy-1,2,3,4-tetrahydro-2,2,4-trimethylchinolin vom Siedepunkt 158 °C bei 0,3 torr (0,4 mbar). Das Produkt erstarrte zu einem weißen Feststoff vom Schmp. 49 °C.

Beispiel 81

Zu einer Lösung von 30 g des Produkts aus Beispiel 80 in 500 ml Toluol wurden 5 g Raney-Nickel gegeben. Im Autoklaven wurden 40 g Ammoniak einkondensiert, die Mischung wurde bei 90 °C und einem Wasserstoffdruck von 300 bar hydriert, bis kein Wasserstoff mehr aufgenommen wurde. Nach dem Abkühlen wurde filtriert und im Wasserstrahlvakuum eingeengt. Das Rohprodukt wurde direkt in Beispiel 82 eingesetzt.

Beispiel 82

31 g des Produkts aus Beispiel 81 und 24,6 g einer 50 %igen wäßrigen Lösung von Tetramethylolacetylendiharnstoff wurden in 200 ml Isobutanol 3 h am Wasserabscheider gekocht. Man engte ein und kristallisierte den Rückstand zweimal aus Acetonitril unter Einsatz von Aktivkohle um. Man erhielt die Verbindung der Formel (XIX) als farblosen Feststoff vom Schmp. 192 °C (Zers.).

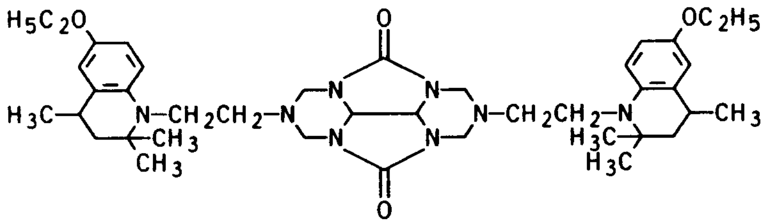

(XIX)

Beispiel 83

65 g 1-Isopropyl-3,3,5,5-tetramethyl-2-piperazinon wurden mit in situ hergestelltem Glykolsäurenitril analog Beispiel 80 52 h gekocht. Die Mischung wurde in 700 ml Aceton eingerührt, der Rückstand wurde abgesaugt und verworfen. Das Filtrat wurde im Wasserstrahlvakuum eingeengt und im Ölpumpenvakuum destilliert. Man erhielt 4-Cyanmethyl-1-isopropyl-3,3,5,5-tetramethyl-2-piperazinon als Flüssigkeit vom Siedepunkt 144 bis 146 °C bei 0,3 torr (0,4 mbar).

Beispiel 84

33 g des Produktes aus Beispiel 83 wurden wie in Beispiel 81 beschrieben hydriert und aufgearbeitet. Das rohe 4-[2-Aminoethyl]-1-isopropyl-3,3,5,5-tetramethyl-2-piperazinon wurde direkt in Beispiel 85 verwendet.

Beispiel 85

22 g des Produktes aus Beispiel 84 und 11,9 g einer 50 %igen wäßrigen Lösung von Tetramethylolacetylendiharnstoff wurden in 75 ml Wasser 3 h auf 80 °C gehalten. Der ausgefallene Niederschlag wurde abgesaugt, getrocknet und aus Essigester umkristallisiert. Man isolierte die Verbindung der Formel (XX) als farblosen Feststoff vom Schmp. 222 °C (Zers.).

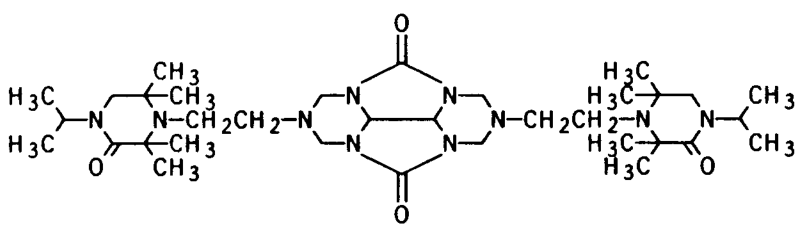

(XX)

Beispiel 86

171 g 2,2,5,5-Tetramethyl-imidazolidin-4-on wurden analog Beispiel 80 10,5 h umgesetzt. Man saugte die Reaktionsmischung ab, fällte mit Essigester unerwünschte Nebenprodukte aus, filtrierte, engte das Filtrat ein und verrührte den Rückstand mit Eiswasser. Man saugte ab, trocknete an der Luft und kristallisierte aus Toluol um. Man erhielt 1-Cyanmethyl-2,2,5,5-tetramethyl-imidazolidin-4-on als farblosen Feststoff vom Schmp. 153 ° C.

Beispiel 87

18 g des Produktes aus Beispiel 86 wurden wie in Beispiel 81 hydriert und aufgearbeitet. Das rohe 1-[2-Aminoethyl]-2,2,5,5-tetramethyl-imidazolidin-4-on konnte direkt in Beispiel 88 eingesetzt werden.

Beispiel 88

18 g des Produktes aus Beispiel 87 und 15,2 g einer 50 %igen wäßrigen Lösung von Tetramethylolacetylendiharnstoff wurden in 150 ml Isobutanol 3,5 h am Wasserabscheider gekocht. Man engte ein, kochte den Rückstand mit Aceton aus und saugte heiß ab. Der Rückstand wurde in 1 l Ethanol-Methanol 1:1 heiß gelöst, mit Aktivkohle versetzt und filtriert. Nach Einengen des Filtrats auf 150 ml fiel die Verbindung der Formel (XXI) aus. Nach Absaugen und Trocknen erhielt man weiße Kristalle vom Schmp. 286 ° C (Zers.).

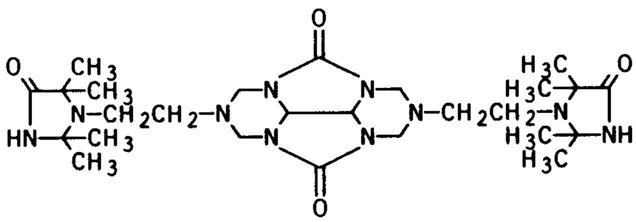

(XXI)

Beispiel 89

54,6 g 3,3-Dimethyl-decahydro-chinoxalin-2-on wurden wie in Beispiel 80 beschrieben mit in situ hergestelltem Glykolsäurenitril 12 h gekocht. Man saugte ab, nahm den Rückstand in 800 ml Dichlormethan auf, filtrierte und engte ein. Der verbliebene Rückstand bestand aus 4-Cyanmethyl-3,3-dimethyl-decahydrochinoxalin-2-on vom Schmp. 198 bis 202 ° C.

Beispiel 90

38 g des Produktes aus Beispiel 89 wurden wie in Beispiel 81 beschrieben hydriert und aufgearbeitet. Das Rohprodukt wurde am Methylcyclohexan umkristallisiert. Man erhielt 4-[2-Aminoethyl]-3,3-dimethyl-decahydrochinoxalin-2-on vom Schmp. 138 bis 146 ° C.

Beispiel 91

23,7 g des Produktes aus Beispiel 90 und 26,2 g einer 50 %igen wäßrigen Lösung von Tetramethylolacetylendiharnstoff wurden in 150 ml iso-Butanol 6 h am Wasserabscheider gekocht. Man saugte bei 80 ° C ab, trocknete und erhielt die Verbindung der Formel (XXII) als farblosen Feststoff vom Schmp. 305 ° C (Zers.).

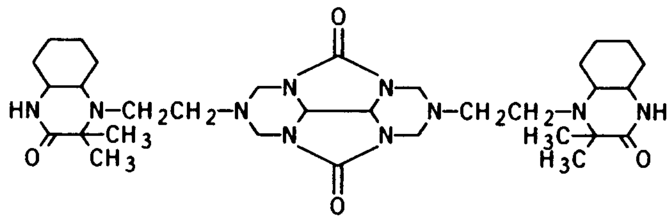

(XXII)

Beispiel 92

169 g 2,2-Pentamethylen-5,5-dimethyl-imidazolidin-4-on wurden analog Beispiel 80 6 h umgesetzt. Die Reaktionsmischung wurde filtriert, eingeengt, der obige Rückstand wurde mit Essigester/Petrolether (1:1) verrührt und filtriert. Das Filtrat wurde eingeengt, der Rückstand aus Toluol umkristallisiert. Man erhielt 1-Cyanmethyl-2,2-pentamethylen-5,5-dimethyl-imidazolidin-4-on vom Schmp. 222 °C (Zers.).

Anwendungsbeispiele

Herstellung der Belichtungsproben aus Polyurethan

Eine Polyolkomponente der Zusammensetzung
41,9 Teile eines Polyetherols der OH-Zahl von 29,0, das durch Addition von Propylenoxid und Ethylenoxid an Propylenglykol erhalten wurde und ungefähr 84 % primäre Hydroxylgruppen besitzt und 42,5 Teile eines Polyetherols der OH-Zahl von 27,0, das durch Addition von Propylenoxid und Ethylenoxid an Trimethylolpropan erhalten wurde und ungefähr 88 % primäre Hydroxylgruppen besitzt und 8,1 Teile 1,4-Butandiol und 1,724 Teile 25 %ige Lösung von Diazabicyclooctan in 1,4-Butandiol und 0,016 Teile Dibutylzinndilaurat und 0,1 Teile des Siliconstabilisators OS 710 der Firma Bayer und 5,49 Teile Frigen 11 und 0,17 Teile Wasser wurde mit der unten angegebenen Stabilisatormischung versetzt und im Mischungsverhältnis 100:48,5 mit einem 23,0 %-isocyanatgruppenhaltigen Prepolymeren bei 25 °C Komponenten- und Werkzeugtemperatur zu Prüfplatten verschäumt. Das NCO-Prepolymer wurde hergestellt aus 87,17 Teilen 4,4'-Diphenylmethandiisocyanat und 4,83 Teilen eines Polyetherols mit der OH-Zahl von 250, das durch Addition von Propylenoxid an Propylenglykol erhalten wurde und 8,0 Teile Dipropylenglykol. Die Proben wurden im Xenotest®450 belichtet und an den Proben der Yellowness Index (YI) gemäß ASTM D 1925 bestimmt. Die Ergebnisse sind in den Anwendungsbeispielen angegeben.

Anwendungsbeispiel 1

| Stabilisator-Mischung | | Konz. (Gew.-%) | Yellowness-Index nach ASTM D 1925 im Xenotest 450 nach 0 h | 48 h | |
|---|---|---|---|---|---|
| Beispiel | 83 | 0,5 | | | |
| Komponente | 1 | 0,5 | 7,35 | 19,64 | |
| Komponente | 2 | 0,25 | | | |
| Komponente | 3 | 0,5 | | | |
| Komponente | 1 | 0,5 | 6,52 | 27,01 | (Vergleich) |
| Komponente | 2 | 0,25 | | | |

Komponente 1 =

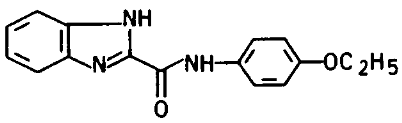

Komponente 2 = Mischung aus α-Tocopherol und Tri(nonylphenyl)-phosphit im Verhältnis 1:10.

Komponente 3 =

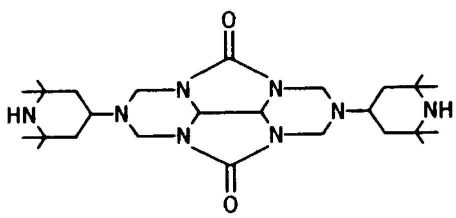

Anwendungsbeispiele 2 und 3

| Stabilisator-Mischung | Konz. (Gew.-%) | Yellowness-Index nach ASTM D 1925 im Xenotest 450 nach | |
|---|---|---|---|
| | | 0 h | 48 h |
| 0-Probe | - | 3,84 | 24,77 |
| (2) | | | |
| Beispiel 88<br>Komponente 1<br>Komponente 2 | 0,5<br>0,5<br>0,25 | 3,89 | 13,56 |
| (3) | | | |
| Beispiel 91<br>Komponente 1<br>Komponente 2 | 0,5<br>0,5<br>0,25 | 3,45 | 12,59 |
| Komponente 4<br>Komponente 5<br>Komponente 2 | 0,5<br>0,5<br>0,25 | 5,03 | 18,18 (Vergleich) |
| Komponente 4<br>Komponente 5<br>Komponente 6 | 0,5<br>0,5<br>0,25 | 5,44 | 17,21 (Vergleich) |
| Komponente 4 = Bis-(1,2,2,6,6-pentamethyl-4-piperidinyl)-sebacat.<br>Komponente 5 = 5'-Methyl-2-(2'-hydroxyphenyl)-benztriazol<br>Komponente 6 = Triethylenglykol-bis-3-(3-t-butyl-4-hydroxy-5-methylphenyl)-propionat. | | | |

## Patentansprüche

**1.** Verwendung von Verbindungen der allgemeinen Formel (I)

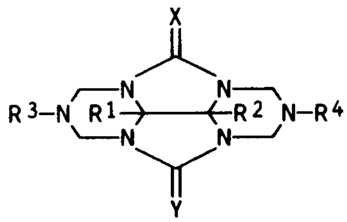

(I),

worin

$R^1$ und $R^2$ unabhängig voneinander Wasserstoff, $C_1$-$C_8$-Alkyl, $C_5$-$C_8$-Cycloalkyl, $C_7$-$C_{12}$-Aralkyl, $C_7$-$C_{14}$-Cycloalkylalkyl, Aryl oder Carbonester bedeuten oder $R^1$ und $R^2$ zusammen eine Tetra-, Penta- oder Hexamethylengruppierung oder einen gegebenenfalls substituierten Rest der Formel

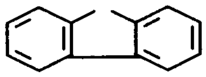

darstellen,

X und Y unabhängig voneinander für Sauerstoff, Schwefel oder $NR^8$ stehen, wobei $R^8$ Wasserstoff, $C_1$-$C_8$-Alkyl oder $C_7$-$C_{12}$-Aralkyl ist, und

$R^3$ und $R^4$ unabhängig voneinander für $C_1$-$C_{22}$-Alkyl, das durch Ethersauerstoff, Schwefel oder Stickstoff unterbrochen sein kann, $C_1$-$C_{22}$-Alkenyl, $C_3$-$C_{12}$-Cycloalkyl, gegebenenfalls substituiertes $C_7$-$C_{12}$-Aralkyl, $C_3$-$C_{12}$-Alkinyl, Hydroxy, einen nichtaromatischen, gegebenenfalls substituierten Heterocyclus, ausgenommen 2,2,6,6-Tetraalkylpiperidinfür den Fall, daß $R^3$ gleich $R^4$ ist, durch einen Heterocyclus substituiertes $C_1$-$C_{22}$-Alkyl, ausgenommen 2,2,6,6-Tetraalkylpiperidin, für den Fall, daß $R^3$ gleich $R^4$ ist, durch Amino, Hydroxy, Chlor, Brom, Jod, Sulfon, Sulfoxid, eine Urethangruppe, eine Harnstoffgruppe, Carboxyl, Carbonester und/oder gegebenenfalls substituiertes Carbamoyl substituiertes $C_1$-$C_{22}$-Alkyl oder für die Gruppierung B-A- stehen, worin -A- ein Brückenglied und B- einen Thiol- oder Cyanrest oder eine Gruppe der Formeln

$-CO_2R^5$ ; 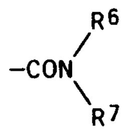 ; $-SO_3R^6$ ; 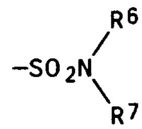 ;

bedeuten, worin

$R^5$ für Wasserstoff, $C_1$-$C_{22}$-Alkyl, $C_5$-$C_8$-Cycloalkyl, $C_7$-$C_{12}$-Cycloalkylalkyl, $C_7$-$C_{12}$-Aralkyl, gegebenenfalls substituiertes Aryl, einen Heterocyclus oder einen Heterocyclus enthaltendes $C_1$-$C_{22}$-Alkyl steht und

$R^6$ und $R^7$ unabhängig voneinander Wasserstoff, $C_1$-$C_{22}$-Alkyl, Ethersauerstoff enthaltendes $C_2$-$C_9$-Alkyl, $C_7$-$C_{12}$-Aralkyl, gegebenenfalls substituiertes Aryl, einen Heterocyclus oder einen Heterocyclus enthaltendes $C_1$-$C_{22}$-Alkyl darstellen oder $R^6$ und $R^7$ gemeinsam mit dem oder den Atom(en), an das (die) sie gebunden sind, ein 3 bis 12-gliedriges Ringsystem bilden,

sowie der Säureadditionssalze und Hydrate dieser Verbindungen, zum Stabilisieren von Kunststoffen und Lacken.

**2.** Verwendung nach Anspruch 1 zum Stabilisieren von Polyolefinen und Polyamiden.

**3.** Verwendung nach Anspruch 1 zum Stabilisieren von Polyurethanen.

**4.** Verwendung nach einem der Ansprüche 1, 2 oder 3 zum Licht- und Wärmeschutz oder als Metallionendesaktivator.

**5.** Verbindungen der allgemeinen Formel (Ib)

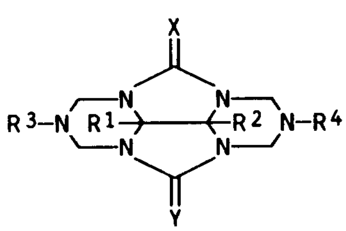

(Ib),

worin

$R^1$ und $R^2$ unabhängig voneinander Methyl oder Phenyl bedeuten und einer der Reste $R^1$ oder $R^2$ auch für Wasserstoff stehen kann oder, wenn X und Y nicht gleichzeitig für Sauerstoff stehen, $R^1$ und $R^2$ auch gleichzeitig Wasserstoff bedeuten können,

X und Y unabhängig voneinander für Sauerstoff, Schwefel oder $NR^8$ stehen, wobei $R^8$ Wasserstoff, $C_1$-$C_8$-Alkyl oder $C_7$-$C_{12}$-Aralkyl ist, und

$R^3$ und $R^4$ unabhängig voneinander für $C_1$-$C_{22}$-Alkyl, das durch Ethersauerstoff, Schwefel oder Stickstoff unterbrochen sein kann, $C_1$-$C_{22}$-Alkenyl, $C_3$-$C_{12}$-Cycloalkyl, gegebenenfalls substituiertes $C_7$-$C_{12}$-Aralkyl, $C_3$-$C_{12}$-Alkinyl, Hydroxy, einen nichtaromatischen, gegebenenfalls substituierten Heterocyclus, ausgenommen 2,2,6,6-Tetraalkylpiperidin für den Fall, daß $R^3$ gleich $R^4$ ist, durch einen Heterocyclus substituiertes $C_1$-$C_{22}$-Alkyl, ausgenommen 2,2,6,6-Tetraalkylpiperidin, für den Fall, daß $R^3$ gleich $R^4$ ist, durch

Amino, Hydroxy, Carboxyl, Carbonester und/oder gegebenenfalls substituiertes Carbamoyl substituiertes $C_1$-$C_{22}$-Alkyl, oder für die Gruppierung B-A- stehen, worin -A- ein Brückenglied und B- einen Thiol- oder Cyanrest oder eine Gruppe der Formeln

$-CO_2R^5$ ; 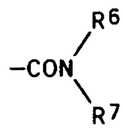 ; $-SO_3R^6$ ; 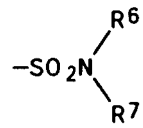 ;

bedeutet, worin

$R^5$ Wasserstoff, $C_1$-$C_{22}$-Alkyl, $C_5$-$C_8$-Cycloalkyl, $C_7$-$C_{12}$-Cycloalkylalkyl, $C_7$-$C_{12}$-Aralkyl, gegebenenfalls substituiertes Aryl, einen Heterocyclus oder einen Heterocyclus enthaltendes $C_1$-$C_{22}$-Alkyl und

$R^6$ und $R^7$ unabhängig voneinander Wasserstoff, $C_1$-$C_{22}$-Alkyl, Ethersauerstoff enthaltendes $C_2$-$C_9$-Alkyl, $C_7$-$C_{12}$-Aralkyl, gegebenenfalls substituiertes Aryl, einen Heterocyclus oder einen Heterocyclus enthaltendes $C_1$-$C_{22}$-Alkyl darstellen oder $R^6$ und $R^7$ gemeinsam mit dem oder den Atom(en), an das (die) sie gebunden sind, ein 3 bis 12-gliedriges Ringsystem bilden,

sowie die Säureadditionssalze und Hydrate dieser Verbindungen.

**6.** Verbindungen der allgemeinen Formel (Ib) nach Anspruch 5, worin X und Y die Bedeutung Sauerstoff besitzen.

**7.** Stabilisierte Kunststoffe und Lacke, enthaltend eine Verbindung der Formeln (I) bzw. (Ib) nach einem der Ansprüche 1, 5 oder 6.

## Claims

**1.** Use of compounds of the general formula (I)

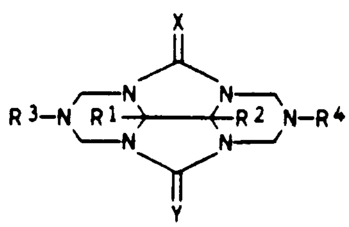

(I)

where

$R^1$ and $R^2$ are each independently of the other hydrogen, $C_1$-$C_8$-alkyl, $C_5$-$C_8$-cycloalkyl, $C_7$-$C_{12}$-aralkyl, $C_7$-$C_{14}$-cycloalkylalkyl, aryl or carboxylic ester, or $R^1$ and $R^2$ are together a tetra- or penta- or hexamethylene group or an optional radical of the formula

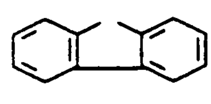

X and Y are each independently of the other oxygen, sulfur or $NR^8$, where $R^8$ is hydrogen, $C_1$-$C_8$-alkyl or $C_7$-$C_{12}$-aralkyl, and

$R^3$ and $R^4$ are each independently of the other $C_1$-$C_{22}$-alkyl, which may be interrupted by etheroxygen, sulfur or nitrogen, $C_1$-$C_{22}$-alkenyl, $C_3$-$C_{12}$-cycloalkyl, substituted or unsubstituted $C_7$-$C_{12}$-aralkyl, $C_3$-$C_{12}$-alkynyl, hydroxyl, a nonaromatic optionally substituted heterocycle, except 2,2,6,6,-tetraalkylpiperidine when $R^3$ is equal to $R^4$, $C_1$-$C_{22}$-alkyl substituted by a heterocycle, except 2,2,6,6-tetraalkylpiperidine when $R^3$ is equal to $R^4$, $C_1$-$C_{22}$-alkyl which is substituted by amino, hydroxyl, chlorine, bromine,

iodine, sulfone, sulfoxide, a urethane or urea group, carboxyl, carboxylate or optionally substituted carbamoyl, or the group B-A-, where -A- is a bridge member and B- is thiol of cyano or a group of the formulae

$-CO_2R^5$ ; 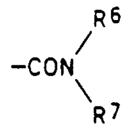 ; $-SO_3R^6$ ; 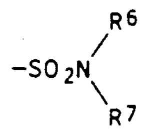 ;

where
$R^5$ is hydrogen, $C_1$-$C_{22}$-alkyl, $C_5$-$C_8$-cycloalkyl, $C_7$-$C_{12}$-cycloalkylalkyl, $C_7$-$C_{12}$-aralkyl, optionally substituted aryl, a heterocycle or $C_1$-$C_{22}$-alkyl which contains a heterocycle, and
$R^6$ and $R^7$ are each independently of the other hydrogen, $C_1$-$C_{22}$-alkyl, etheroxygen-containing $C_2$-$C_9$-alkyl, $C_7$-$C_{12}$-aralkyl, optionally substituted aryl, a heterocycle or $C_1$-$C_{22}$-alkyl which contains a heterocycle, or $R^6$ and $R^7$ together with the atom(s) to which they are bonded form a 3- to 12-membered ring system,

and also of acid addition salts and hydrates of these compounds, for stabilizing plastics and surface coatings.

**2.** Use as claimed in claim 1 for stabilizing polyolefins and polyamides.

**3.** Use as claimed in claim 1 for stabilizing polyurethanes.

**4.** Use as claimed in any one of claims 1, 2 and 3, for light and heat stabilization or as a metal ion deactivator.

**5.** Compounds of the general formula (Ib)

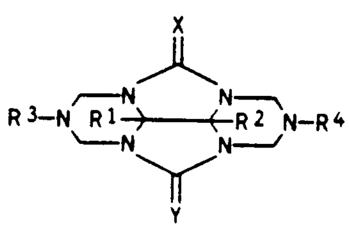

(Ib)

where

$R^1$ and $R^2$ are each independently of the other methyl or phenyl and one of $R^1$ and $R^2$ may also be hydrogen or, unless X and Y are both oxygen, $R^1$ and $R^2$ may both be hydrogen,

X and Y are each independently of the other oxygen, sulfur or $NR^8$, where $R^8$ is hydrogen, $C_1$-$C_8$-alkyl or $C_7$-$C_{12}$-aralkyl, and

$R^3$ and $R^4$ are each independently of the other $C_1$-$C_{22}$-alkyl, which may be interrupted by etheroxygen, sulfur or nitrogen, $C_1$-$C_{22}$-alkenyl, $C_3$-$C_{12}$-cycloalkyl, optionally substituted $C_7$-$C_{12}$-aralkyl, $C_3$-$C_{12}$-alkynyl, hydroxyl, a nonaromatic optionally substituted heterocycle, except 2,2,6,6-tetraalkylpiperidine if $R^3$ is equal to $R^4$, $C_1$-$C_{22}$-alkyl substituted by a heterocycle, except 2,2,6,6-tetraalkylpiperidine if $R^3$ is equal to $R^4$, amino-, hydroxyl-, carboxyl-, carboxylate- and/or optionally substituted carbamoyl-substituted $C_1$-$C_{22}$-alkyl or the group B-A-, where -A- is a bridge member and B- is thiol, cyano or a group of the formulae

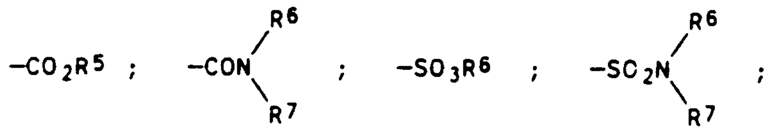

where
$R^5$ is hydrogen, $C_1$-$C_{22}$-alkyl, $C_5$-$C_8$-cycloalkyl, $C_7$-$C_{12}$-cycloalkylalkyl, $C_7$-$C_{12}$-aralkyl, optionally substituted aryl, a heterocycle or $C_1$-$C_{22}$-alkyl containing a heterocycle, and
$R^6$ and $R^7$ are each independently of the other hydrogen, $C_1$-$C_{22}$-alkyl, etheroxygen-containing $C_2$-$C_9$-alkyl, $C_7$-$C_{12}$-aralkyl, optionally substituted aryl, a heterocycle or $C_1$-$C_{22}$-alkyl containing a heterocycle, or $R^6$ and $R^7$ together with the atom(s) to which they are bonded form a 3- to 12-membered ring system, and the acid addition salts and hydrates of these compounds.

6. Compounds of the general formula (Ib) as claimed in claim 5, wherein X and Y are each oxygen.

7. Stabilized plastics or surface coatings containing a compound of the formulae (I) or (Ib) as claimed in any one of claims 1, 5 and 8.

## Revendications

1. Utilisation de composés de la formule générale (I)

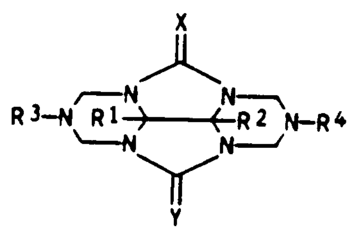

(I),

dans laquelle

$R^1$ et $R^2$ représentent chacun, indépendamment l'un de l'autre, un atome d'hydrogène, un radical alkyle en $C_1$-$C_8$, cycloalkyle en $C_5$-$C_8$, aralkyle en $C_7$-$C_{12}$, alkylcycloalkyle en $C_7$-$C_{14}$, aryle ou ester carboxylique, ou bien $R^1$ et $R^2$ représentent ensemble un groupement tétra-, penta- ou hexaméthylène, ou un radical, éventuellement substitué, de la formule

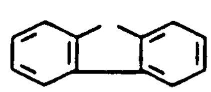

X et Y représentent chacun, indépendamment l'un de l'autre, un atome d'oxygène, un atome de soufre ou le radical $NR^8$ où $R^8$ représente un atome d'hydrogène, un radical alkyle en $C_1$-$C_8$, ou aralkyle en $C_7$-$C_{12}$ et

$R^3$ et $R^4$ représentent chacun, indépendamment l'un de l'autre, un radical alkyle en $C_1$-$C_{22}$, qui peut être interrompu par de l'oxygène éthéré, du soufre ou de l'azote, alcényle en $C_1$-$C_{22}$, cycloalkyle en $C_3$-$C_{12}$, aralkyle en $C_7$-$C_{12}$ éventuellement substitué, alcynyle en $C_3$-$C_{12}$, hydroxyle, un noyau hétérocyclique, non aromatique, éventuellement substitué, à l'exception de la 2,2,6,6-tétraalkylpipéridine dans le cas où $R^3$ est identique à $R^4$, alkyle en $C_1$-$C_{22}$ substitué par un noyau hétérocyclique, à l'exception de la 2,2,6,6-tétraalkylpipéridine au cas où $R^3$ est identique à $R^4$, alkyle en $C_1$-$C_{22}$ substitué par un atome de chlore, de brome, d'iode, un radical amino, hydroxyle, sulfone, sulfoxyde, uréthanne, urée, carboxyle, ester carboxylique et/ou carbamoyle éventuellement substitué, ou le groupement B-A- où -A- représente un élément de pontage et B- représente un radical thiol ou cyano, ou un groupe des formules

$-CO_2R^5$ , 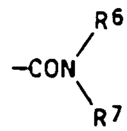 , $-SO_3R^6$ , 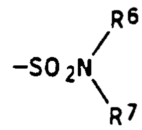 ,

où

$R^5$ représente un atome d'hydrogène, un radical alkyle en $C_1$-$C_{22}$, cycloalkyle en $C_5$-$C_8$, alkylcycloalkyle en $C_7$-$C_{12}$, aralkyle en $C_7$-$C_{12}$, aryle éventuellement substitué, un noyau hétérocyclique ou un radical alkyle en $C_1$-$C_{22}$ contenant un noyau hétérocyclique et

$R^6$ et $R^7$ représentent chacun, indépendamment l'un de l'autre, un atome d'hydrogène, un radical alkyle en $C_1$-$C_{22}$, un radical alkyle en $C_2$-$C_9$ contenant de l'oxygène éthéré, un radical aralkyle en $C_7$-$C_{12}$, un radical aryle éventuellement substitué, un noyau hétérocyclique ou un radical alkyle en $C_1$-$C_{22}$ contenant un noyau hétérocyclique, ou bien

$R^6$ et $R^7$ représentent, communément avec le ou les atomes auxquels ils sont liés, un système cyclique à 3-12 chaînons,

comme aussi des sels d'addition d'acides et des hydrates de ces composés, pour la stabilisation de matières plastiques et de laques ou peintures.

**2.** Utilisation suivant la revendication 1 en vue de la stabilisation de polyoléfines et de polyamides.

**3.** Utilisation suivant la revendication 1 en vue de la stabilisation de polyuréthannes.

**4.** Utilisation suivant l'une quelconque des revendications 1, 2 et 3, en vue de la protection contre la lumière et la chaleur, ou à titre de désactivateur d'ions de métaux.

**5.** Composés de la formule générale (Ib)

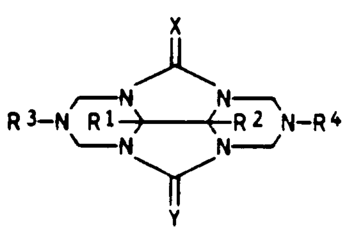

(Ib),

dans laquelle

$R^1$ et $R^2$ représentent chacun, indépendamment l'un de l'autre, un radical méthyle ou phényle et l'un des symboles $R^1$ et $R^2$ peut aussi représenter un atome d'hydrogène, ou bien lorsque X et Y ne représentent pas simultanément de l'oxygène, $R^1$ et $R^2$ peuvent aussi représenter simultanément de l'oxygène,

X et Y représentent chacun, indépendamment l'un de l'autre, de l'oxygène, du soufre ou le radical $NR^8$ où $R^8$ représente un atome d'hydrogène, un radical alkyle en $C_1$-$C_8$ ou aralkyle en $C_7$-$C_{12}$ et

$R^3$ et $R^4$ représentent chacun, indépendamment l'un de l'autre, un radical alkyle en $C_1$-$C_{22}$, qui peut être interrompu par de l'oxygène éthéré, du soufre ou de l'azote, un radical

alcényle en $C_1$-$C_{22}$, cycloalkyle en $C_3$-$C_{12}$, aralkyle en $C_7$-$C_{12}$ éventuellement substitué, alcynyle en $C_3$-$C_{12}$, hydroxyle, un noyau hétérocyclique non aromatique, éventuellement substitué, à l'exception de la 2,2,6,6-tétraalkylpipéridine dans le cas où $R^3$ est identique à $R^4$, alkyle en $C_1$-$C_{22}$ substitué par un noyau hétérocyclique, à l'exception de la 2,2,6,6-tétraalkylpipéridine au cas ou $R^3$ est identique à $R^4$, alkyle en $C_1$ à $C_{22}$ susbstitué par un radical amino, hydroxyle, carboxyle, ester carboxylique et/ou carbamoyle éventuellement substitué, ou le groupement B-A- où -A représente un élément formant pont et B- représente un reste thiol ou cyano, ou un groupe des formules

$-CO_2R^5$ ; 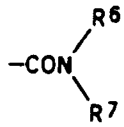 , $-SO_3R^6$ , 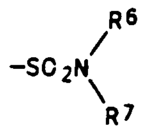 ,

dans lesquelles

$R^5$ représente un atome d'hydrogène, un radical alkyle en $C_1$-$C_{22}$, cycloalkyle en $C_5$-$C_8$, alkylcycloalkyle en $C_7$-$C_{12}$, aralkyle en $C_7$-$C_{12}$,aryle éventuellement substitué, un noyau hétérocyclique ou un radical alkyle en $C_1$-$C_{22}$ contenant un hétérocycle et

$R^6$ et $R^7$ représentent chacun indépendamment l'un de l'autre, un atome d'hydrogène, un radical alkyle en $C_1$-$C_{22}$, alkyle en $C_2$-$C_9$ contenant de l'oxygène éthéré, aralkyle en $C_7$-$C_{12}$, aryle éventuellement substitué, un noyau hétérocyclique ou un radical alkyle en $C_1$-$C_{22}$ contenant un noyau hétérocyclique, ou bien

$R^6$ et $R^7$ représentent, communément avec le ou les atomes auxquels ils sont attachés, un système cyclique à 3 à 12 chaînons,

comme aussi les sels d'addition d'acides et les hydrates de ces composés.

**6.** Composés de la formule générale (Ib), suivant la revendication 5, dans laquelle X et Y représentent de l'oxygène.

**7.** Laques ou peintures et matières plastiques stabilisées, qui contiennent un composé de la formule (I) ou de la formule (Ib) suivant l'une quelconque des revendications 1, 5 et 6.